# NEW EUROPEAN PATENT SPECIFICATION

(11) **EP 0 742 902 B2**
(45) Date of publication and mention of the opposition decision: **13.06.2007**
(45) Mention of the grant of the patent: 21.11.2001
(21) Application number: 94926817.1
(22) Date of filing: 19.09.1994
(51) Int. Cl.: G01N 33/53, G01N 33/68, C07K 14/78

(54) **A METHOD OF ASSAYING COLLAGEN FRAGMENTS IN BODY FLUIDS, A TEST KIT AND MEANS FOR CARRYING OUT THE METHOD AND USE OF THE METHOD TO DIAGNOSE THE PRESENCE OF DISORDERS ASSOCIATED WITH THE METABOLISM OF COLLAGEN**
EIN VERFAHREN ZUR BESTIMMUNG VON KOLLAGENFRAGMENTEN IN KÖRPERFLÜSSIGKEITEN, EIN TESTKIT UND MITTEL ZUR DURCHFÜHRUNG DES VERFAHRENS, UND SEINE VERWENDUNG ZUR FESTSTELLUNG VON MIT DEM KOLLAGENMETABOLISMUS ASSOZIIERTEN STÖRUNGEN
PROCEDE DE DOSAGE DE FRAGMENTS DE COLLAGENE DANS LES LIQUIDES BIOLOGIQUES, MATERIEL DE TEST ET MOYENS POUR APPLIQUER CE PROCEDE, ET UTILISATION DE CE PROCEDE POUR DIAGNOSTIQUER DES TROUBLES ASSOCIES AU METABOLISME DU COLLAGENE

(30) Priority: 17.09.1993 DK 104093
(43) Date of publication of application: 20.11.1996
(60) Divisional application: 01101773.8
(73) Proprietor: Osteometer Biotech AS, 2730 Herlev (DK)
(72) Inventor: QVIST, Per, DK-2930 Klampenborg (DK); BONDE, Martin, DK-2800 Lyngby (DK)
(74) Representative: Smart, Peter John
(86) International application number: PCT/DK1994/000348
(87) International publication number: WO 1995/008115

(56) References cited:
- EP-A- 0 505 210
- WO-A-89/04491
- WO-A-91/08478
- WO-A-94/03813
- WO-A-94/14844
- US-A- 4 504 587
- US-A- 5 817 755
- DIALOG INFORMATION SERVICES, File 154, Dialog Accession No. 08392768, HANSON DA et al.: "A Specific Immunoassay for Monitoring Human Bone Resorption:Quantitation of Type I Collagen Cross-Linked N-Telopeptides in Urine"; & J BONE MINER RES (UNITED STATES), Nov 1992, 7 (11), p. 1251-8.
- Timpl et al, the Journal of Immunology, 1972, 108 : 119-125
- DIALOG INFORMATION SERVICES, File 154, Dialog Accession No. 08392768, HANSON DA et al.: "A Specific Immunoassay for Monitoring Human Bone Resorption: Guantitat of Type I Collagen Cross-Linked N-Telopeptides in Urine"; & J BONE MINER RES (UNITED STATES), Nov 1992, 7 (11), p. 1251-8.

## Description

The present invention relates to determining collagen fragments in body fluids. This is relevant to the diagnosis of the presence of disorders associated with the metabolism of collagen, especially osteoporosis.

### BACKGROUND OF THE INVENTION

### Collagens and Disorders of Collagen Metabolism

Osteoporosis is the most common bone disease in humans. Primary osteoporosis, accompanied by increased susceptibility to fractures, results from a progressive reduction in skeletal bone mass. It is estimated to affect 15-20 million individuals in the USA alone. Its basis is an age-dependent imbalance in bone remodelling, i.e. in the rates of formation and resorption of bone tissue.

In the USA about 1.2 million osteoporosis-related fractures occur in the elderly each year including about 538,000 compression fractures of the spine, about 227,000 hip fractures and a substantial number of early fractured peripheral bones. Between 12 and 20% of the hip fractures are fatal because they cause sever trauma and bleeding, and half of the surviving patients require nursing home care. Total costs from osteoporosis-related injuries now amount to at least $10 billion annually in the USA (Riggs, New England Journal of Medicine, 327:620-627 (1992)).

Osteoporosis is most common in postmenopausal women who, on average, lose 15% of their bone mass in the 10 years after menopause. This disease also occurs in men as they get older and in young amenorrheic women athletes. Despite the major, and growing, social and economic consequences of osteoporosis, the availability of reliable assays for measuring bone resorption rates in patients or in healthy subjects is very limited. Other disorders entailing (and correlated with) abnormalities in collagen metabolism include Paget's disease, Marfan's syndrome, osteogenesis imperfecta neoplastic growth in collagenous tissue, dwarfism, rheumatoid arthritis, osteoarthritis and vasculitis syndrome.

Three known classes of human collagen have been described to date. The Class I collagens, subdivided into types I, II, III, V, and XI, are known to form fibrils. The aminoacid sequence of type I-III (to the extent it has been elucidated) is attached as Appendix A.

Collagen type I accounts for more than 90% of the organic matrix of bone. Therefore, in principle, it is possible to estimate the rate of bone resorption by monitoring the degradation of collagen type I. Likewise, a number of other disease states involving connective tissue can be monitored by determining the degradation of collagen. Examples are collagen type II degradation associated with rheumatoid arthritis and osteoarthritis and collagen type III degradation in vasculitis syndrome.

Amino acid sequences of human type III collagen, human pro α1(II) collagen, and the entire prepro α1(III) chain of human type III collagen and corresponding cDNA clones have been investigated and determined by several groups of researchers; see Loil et al., Nucleic Acids Research 12:9383-9394 (1984): Sangiorgi et al., Nucleic Acids Research, 13:2207-2225 (1985); Baldwin et al., Biochem J., 262:521-528 (1989); and Ala-Kokko et al., Biochem. J., 260:509-516 (1989).

Type I, II, and III collagens are all formed in the organism as procollagen molecules, comprising N-terminal and C-terminal propeptide sequences, which are attached to the core collagen molecules. After removal of the propeptides, which occur naturally in vivo during collagen synthesis, the remaining core of the collagen molecules consists largely of a triple-helical domain having terminal telopeptide sequences which are non-triple-helical. These telopeptide sequences have an important function as sites of intermolecular cross-linking of collagen fibrils extracellularly. The alpha-helical region also includes crosslinkable sites. Peptides from this region are part of the present invention.

Intermolecular cross-links provide collagen fibrils with biomechanical stability. The formation of these cross-links is initiated by modification of lysine and hydroxylysine residues to the corresponding aldehydes. Several of these residues located on adjacent chains of collagen will spontaneously form different intermolecular cross-links. The exact position of the sites for cross-linking on collagen telopeptides and from the helical region has been previously described. See, for example, Kühn, K., in Immunochemistry of the extracellular matrix, 1:1-29, CRC Press, Inc., Boca Raton, Florida (1982), Eyre, D.R., Ann.Rev. Biochem., 53:717-48 (1984) or US patent No. 5 140 103). Furthermore, the amino acid sequences of some potential sites for cross-linking in type I, II, and III collagen are given in Table 1 below.

The fibrous proteins, collagen and elastin, are cross-linked by a unique mechanism based on aldehyde formation from lysine or hydroxylysine side chains. Four homologous loci of cross-linking are evident in molecules of type I, II and III collagens (for review see Kühn, K., in Immunochemistry of the extracellular matrix, 1:1-29 (1982)). Two are aldehyde sites, one in each telopeptide region. The other two sites are hydroxylysine symmetrically placed at about 90 residues from each end of the molecule. When collagen molecules pack into fibrils, these latter sites in the helical region align and react with telopeptide aldehydes in adjacent molecules. There is now strong evidence that 3- hydroxypyridinium residues are the mature cross-link coming from hydroxylysine-derived aldehydes. The mature cross-linking residues of the other pathway, i.e. from aldehyde formation of lysine residues, is, however, still unknown.

### Prior Art Assays for Collagen Degradation

In the past, assays have been developed for monitoring degradation of collagen in vivo by measuring various biochemical markers, some of which have been degradation products of collagen. However, none of these methods are based upon the use of immunological binding partners in the form of antibodies which are immunoreactive with synthetic peptides having a sequence essentially derived from collagen fragments having crosslinkable sites.

For example, hydroxyproline, an amino acid largely restricted to collagen, and the principal structural protein in bone and all other connective tissues, is excreted in urine. Its excretion rate is known to be increased in certain conditions, notably Paget's disease, a metabolic bone disorder in which bone turnover is greatly increased, as discussed further below.

For this reason, urinary hydroxyproline has been used extensively as an amino acid marker for collagen degradation; Singer, F.R. et al., Metabolic Bone Disease, Vol. II (eds. Avioli, L.V., and Kane, S.M.), 489-575 (1978), Academic Press, New York.

US patent No. 3,600,132 discloses a process for the determination of hydroxyproline in body fluids such as serum, urine, lumbar fluid and other intercellular fluids in order to monitor deviations in collagen metabolism. The patent states that hydroxyproline correlates with increased collagen anabolism or catabolism associated with pathological conditions such as Paget's disease, Marfan's syndrome, osteogenesis imperfecta, neoplastic growth in collagen tissues and in various forms of dwarfism.

Bone resorption associated with Paget's disease has also been monitored by measuring small peptides containing hydroxyproline, which are excreted in the urine following degradation of bone collagen; Russell et al., Metab. Bone Dis. and Rel. Res. 4 and 5, 255-262 (1981), and Singer, F.R., et al., supra.

In the case of Paget's disease, the increased urinary hydroxyproline probably comes largely from bone degradation; hydroxyproline, however, generally cannot be used as a specific index for bone degradation. Much of the hydroxyproline in urine may come from new collagen synthesis (considerable amounts of the newly made protein are degraded and excreted without ever becoming incorporated into tissue fabric), and from turnover of certain blood proteins as well as other proteins that contain hydroxyproline.

Furthermore, about 80% of the free hydroxyproline derived from protein degradation is metabolized in the liver and never appears in the urine. Kiviriko, K.I., Int. Rev. Connect. Tissue Res. 5:93 (1970), and Weiss, P.H. and Klein, L., J. Clin. Invest. 48:1 (1969). Hydroxyproline is a good marker for osteoporosis, but it is troublesome to handle. It is specific for collagen in bones.

Hydroxylysine and its glycoside derivatives, both peculiar to collagenous proteins, have been considered to be more accurate than hydroxyproline as markers of collagen degradation. However, for the same reasons described above for hydroxyproline, hydroxylysine and its glycosides are probably equally non-specific markers of bone resorption; Krane, S.M. and Simon, L.S., Develop. Biochem. 22:185 (1981).

Other researchers have measured the cross-linking compound 3-hydroxypyridinium in urine as an index of collagen degradation in joint diseases. See, for background and as examples, Wu and Eyre, Biochemistry, 23:1850 (1984): Black et al., Annals of the Rheumatic Diseases, 48:641-644 (1989); Robins et al.; Annals of the Rheumatic Diseases, 45:969-973 (1986); and Seibel et al., The Journal of Dermatology, 16:964 (1989). In contrast to the present invention, these prior researchers have hydrolyzed peptides from body fluids and then looked for the presence of individual 3-hydroxypyridinium residues.

Assays for determination of the degradation of type I, II, and III collagen are disclosed in US patent No. 4,973,666 and US patent No. 5,140,103. However, both these patents are restricted to collagen fragments containing the crosslinker 3-hydroxypyridinium, whereas the present invention does not rely on the presence or absence of this particular cross-linking structure. Furthermore, the above-mentioned assays require tedious and complicated purifications from urine of collagen fragments containing 3-hydroxypyridinium to be used for the production of antibodies and for antigens in the assays.

At present very few clinical data using the approach described in US patent No. 4,973,666 and US patent No. 5,140,103 are available. Particularly, no data concerning the correlation between the urinary concentration (as determined by methods described in the above-mentioned patents) of 3-hydroxypyridinium containing telopeptides of type I collagen and the actual bone loss (as determined by repeated measurements by bone densiometry) are published. The presence of 3-hydroxypyridinium containing telopeptides in urine requires the proper formation in bone tissue of this specific cross-linking structure at various times before the bone resorbing process. Very little information on these processes is available and the present invention seeks to avoid this dependence of the correct formation of the cross-linking structure. Furthermore, preliminary data indicate that in one embodiment of the present invention a major fraction of the molecules reactive in the assay has a molecular weight of more than 4.000 daltons. On the contrary, only molecules with a molecular weight below 2.000 daltons are identified in urine with the monoclonal antibody used in the assay; Hanson et al., Journal of Bone and Mineral Research 7: 1251-1258 (1992). This demonstrates that the method according to the present invention has a very different profile of reactivities, i.e. it detects very different molecules, compared to methods described in the two above-mentioned US patents.

GB patent application No. 2,205,643 reports that the degradation of type III collagen in the body can be quantitatively determined by measuring the concentration of an N-terminal telopeptide from type III collagen in a body fluid. This method does not relate to methods employing antibodies reactive with specific, low molecular weight sequences around crosslinkable structures. Instead, the method uses antibodies generated to N-terminal telopeptides released by bacterial collagenase degradation of type III collagen, said telopeptides being labelled and used in the assay.

Schröter-Kermani et al., Immunol. Invest. 19:475-491 (1990) describe immunological measurement systems based on CNBr fragments of collagen type I and II. Use is made of pepsin-solubilized collagen, leaving the telopeptides in the tissue (cf. the above-mentioned GB patent application No. 2,205,643). There is therefore no conformity between the fragments and the antibodies raised therefrom. Further, the reference only describes measurements on extracted tissue samples.

The development of a monoclonal antibody raised against pepsin-solubilized type I collagen is described in Werkmeister et al., Eur. J. Biochem. 187:439-443 (1990). The antibody is used for immunohistochemical staining of tissue segments and for measuring the collagen content in cell cultures. The measurements are not carried out on body fluids.

EP patent application No. 0 505 210 describes the development of antibody reagents on a C-terminal telopeptide from type I collagen. The immunogen is prepared by solubilizing human bone collagen with bacterial collagenase. The antibodies thus prepared are able to react with both cross-linked and non-cross-linked telopeptides, and cross-linkers other than pyridinoline may be used. However, the method leads to an immunoassay which is markedly different from the assay according to the present invention.

International patent application No. WO 91/09114 discloses certain synthetic peptides which are used to promote cellular adhesion to a solid substrate. The use of the synthetic peptides as immunological reagents is not mentioned.

There are a number of reports indicating that collagen degradation can be measured by quantitating certain procollagen peptides. Propeptides are distinguished from telopeptides and alpha-helical region of the collagen core by their location in the procollagen molecule and the timing of their cleavage in vivo; see US patent No. 4,504,587; US patent No. 4,312,853; Pierard et al., Analytical Biochemistry 141:127-136 (1984); Niemela, Clin. Chem. 31/8:1301-1304 (1985); and Rohde et al., European Journal of Clinical Investigation, 9:451-459 (1979).

EP patent application No. 0 298 210 and No. 0 339 443 both describe immunological determination of procollagen peptide type III and fragments thereof. Further, a method based on the measurement of procollagen is disclosed in EP patent application No. 0 465 104. Since the formation of the procollagen peptides and the formation of the collagen fragments take place at different times and since the fragments originate from different parts of the collagen molecule, these methods are clearly different from the method of the invention.

The use of synthetic peptides with sequences derived from type IX collagen for the development of immunological reagents is disclosed in PCT patent application No. WO 90/08195. Likewise the application describes the use of the antibodies thus produced for the determination of type IX collagen fragments in body fluids. Since type IX collagen contains no crosslinkable sites, this patent application does not anticipate the present invention.

US patent No. 4,778,768 relates to a method of determining changes occurring in articular cartilage involving quantifying proteoglycan monomers or antigenic fragments thereof in a synovial fluid sample. This US patent does not relate to detecting collagen fragments derived from degraded collagen.

Dodge, J. Clin Invest 83:647-661 (1981) discloses methods for analyzing type II collagen degradation utilizing a polyclonal antiserum that specifically reacts with unwound alpha-chains and cyanogen bromide-derived peptides of human and bovine type II collagens. Contrary to the present invention the degradation products of collagen were not detected in a body fluid, but histochemically by staining of cell cultures, i.e. by "in situ" detection. The main difference between Dodge and the present invention is that Dodge measures type II collagen degradation in situ.

None of the above references specify the structures of particular telopeptide or alpha-helical degradation products that could be measured to determine the amount of degraded fibrillar collagen in vivo.

WO-A-9403813 describes a competitive immunoassay for detecting collagen or collagen fragments in a sample wherein a binding partner containing a synthetic linear peptide corresponding to the non-helical C-terminal or N-terminal domain of collagen is incubated with an antibody to the linear synthetic peptide and the sample, and wherein the binding of the antibody to the binding partner is determined.

WO-A-9414844 discloses C-telopeptide assays based on a lengthy synthetic peptide. It was not published before the priority date hereof.

WO-A-9403813 and WO-A-9414844 represent prior art within the meaning of Art. 54(3) EPC.

### SUMMARY OF THE INVENTION

The method of the present invention may be used for determination of the degradation of human collagen of Type I.

The present invention now provides a method of assay for collagen Type I fragments in a body fluid, in which collagen fragments in a said body fluid and a synthetic peptide having a sequence derived from collagen and having a potential site for cross-linking but containing no collagen cross-link structure immobilised on a solid surface are contacted with an immunological binding partner which is immunoreactive with the immobilised synthetic peptide and with collagen fragments in said body fluid, wherein the immunological binding partner has been raised against a peptide consisting of the amino acid sequence Gln-Tyr-Asp-Gly-Lys-Gly-Val-Gly and is immunoreactive with an immobilised peptide consisting of the amino acid sequence Gln-Tyr-Asp-Gly-Lys-Gly-Val-Gly.

The invention includes a method of assaying Type I collagen fragments in a body fluid, comprising:
(a) contacting a sample of the body fluid with a synthetic peptide immobilised on a solid support and with an immunological binding partner which is immunologically reactive with said immobilised synthetic peptide, and which has been raised against a peptide consisting of the amino acid sequence Gln-Tyr-Asp-Gly-Lys-Gly-Val-Gly, wherein the collagen fragments compete with said synthetic peptide for binding with the immunological binding partner; and
(b) quantifying the amount of collagen fragments in the body fluid by measuring the amount of binding of said immunological binding partner with said synthetic peptide;
wherein
said immunological binding partner is immunologically reactive with a peptide immobilised on a solid support and consisting of the amino acid sequence Gln-Tyr-Asp-Gly-Lys-Gly-Val-Gly; and said immobilised synthetic peptide is derived from collagen, contains no collagen cross-link structure but contains a potential site for cross linking, and is immunologically reactive with said immunological binding partner.

Said immobilised synthetic peptide may consist of fewer than 10 amino acids.

The immobilised synthetic peptide may have the amino acid sequence Gln-Tyr-Asp-Gly-Lys-Gly-Val-Gly or may have said sequence Gln-Tyr-Asp-Gly-Lys-Gly-Val-Gly modified by the addition or omission of one or more amino acids whilst retaining the ability to raise antibodies recognising the corresponding native collagen fragment or to inhibit the binding of such antibodies to the corresponding native collagen fragment.

Said immunological binding partner may be a whole antibody or may be an antibody fragment.

Said antibody may be a monoclonal antibody.

A peptide may be used containing no collagen cross-link structure but having a potential site for cross-linking in a method for measuring Type I collagen fragments in a sample, wherein the peptide consists of the sequence Gln-Tyr-Asp-Gly-Lys-Gly-Val-Gly or consists of said sequence Gln-Tyr-Asp-Gly-Lys-Gly-Val-Gly modified by the addition or omission of one or more amino acids whilst retaining the ability to raise antibodies recognising the corresponding native collagen fragment or to inhibit the binding of such antibodies to the corresponding native collagen fragment.

The peptide is immobilised to a solid support.

A comparison may be made of the detected collagen fragments to those of a predetermined standard developed by measuring the same class of collagen fragments in normal individuals, i.e. individuals not afflicted with a disorder affecting collagen metabolism, said individuals being sex- and age-matched with the subjects being tested.

In the use of the described kits competitive binding takes place of collagen fragments in body fluids and of the synthetic peptide.

For purposes of the present invention, as disclosed and claimed herein, the following terms are as defined below:
"Antibody": A monoclonal or polyclonal antibody or immunoreactive fragment thereof (i.e. capable of binding the same antigenic determinant), including - but not limited to - Fab, Fab', and F(ab')2 fragments.
"Test kit": A combination of reagents and instructions for use in conducting an assay.
"Essentially derived (about structures)": Structures with similar antigenicity, i.e. with an ability, above the level of a non-related peptide, to inhibit the binding of any of the mentioned synthetic peptides to an immunological binding partner immunoreactive with said synthetic peptide.
"CrossLaps ELISA": Competitive immunoassay based on the reactivity of an antiserum to the α1(I)C1 amino acid sequence EKAHDGGR. This assay is also named 8AA ELISA.

It is contemplated that the method may also be used for assaying collagen fragments in animal body fluids, e.g. for determination of the collagen metabolism. It also can be used during clinical testing of new drugs to assess the impact of these drugs on collagen metabolism.

We describe here methods for assaying collagen fragments by the use of synthetic peptides corresponding to the above mentioned sequence of collagen. Generally, these synthetic peptides will have fewer than 10 amino acids. Also, the synthetic peptides have a potential site for cross-linking at the lysine incorporated in the structure.

Antibodies for use in the invention include monoclonal antibodies (as well as binding fragments thereof, e.g. Fab) coupled to a detectable marker. Examples of detectable markers include, but are not limited to, enzymes, chromophores, fluorophores, coenzymes, enzyme inhibitors, chemiluminescent materials, paramagnetic metals, spin labels and radioisotopes.

In a representative assay, collagen fragments in the patient's body fluid and a synthetic peptide immobilised on a solid surface are contacted with an immunological binding partner which is immunoreactive with the synthetic peptide. Suitable body fluids are e.g. human urine, blood, serum, plasma and synovial fluid. It is contemplated that the method may also be used e.g. on saliva and sweat. The body fluid may be used as it is, or it may be purified prior to the contacting step. This purification step may be accomplished using a number of standard procedures, including, but not limited to, cartridge adsorption and elution, molecular sieve chromatography, dialysis, ion exchange, alumina chromatography, hydroxyapatite chromatography, and combinations thereof.

Synthetic peptides may be used for the raising of antibodies and subsequently incorporated in the assay for quantitation of collagen fragments generated in vivo by collagen degradation. The synthetic peptides may therefore also be characterised as immunogenic agents and may be used in immunogenic compositions.

We describe herein test kits useful for quantitating in a body fluid the amount of collagen fragment derived from the degradation of collagen. The kits comprise at least one immunological binding partner, e.g. a monoclonal or polyclonal antibody specific for a peptide derived from the degradation of collagen. If desired, the immunological binding partner of the test kit may be coupled to detectable markers such as the ones described above. Generally speaking, the immunological binding partner is therefore also useful as a diagnostic agent.

The invention is described in more detail below. Reference is made to the appended drawings.

### BRIEF DESCRIPTION OF THE DRAWINGS

Figures 1, 2 and 3 are typical standard curves for the α1(I)Cl immunoassay (also called "CrossLaps") (Fig. 1), the α1(I)N1 immunoassay (Fig.2) and the α2(I)N1 immunoassay (Fig.3) to be described in more detail in the examples;
Figure 4 shows the correlation between total pyridinoline (HPLC) and the CrossLaps immunoassay;
Figure 5 shows the correlation between total pyridinoline (HPLC) and the α1(I)N1 immunoassay;
Figure 6 shows the eptipe specificity of the antibody MabA7;
Figure 7 shows the correlation between the CrossLaps ELISA based on the α1(I)C1 peptide fragment and an MabA7 ELISA;
Figure 8 shows the correlation between the CrossLaps ELISA and an Nα2 ELISA based on the α2(I)N1 peptide fragment,

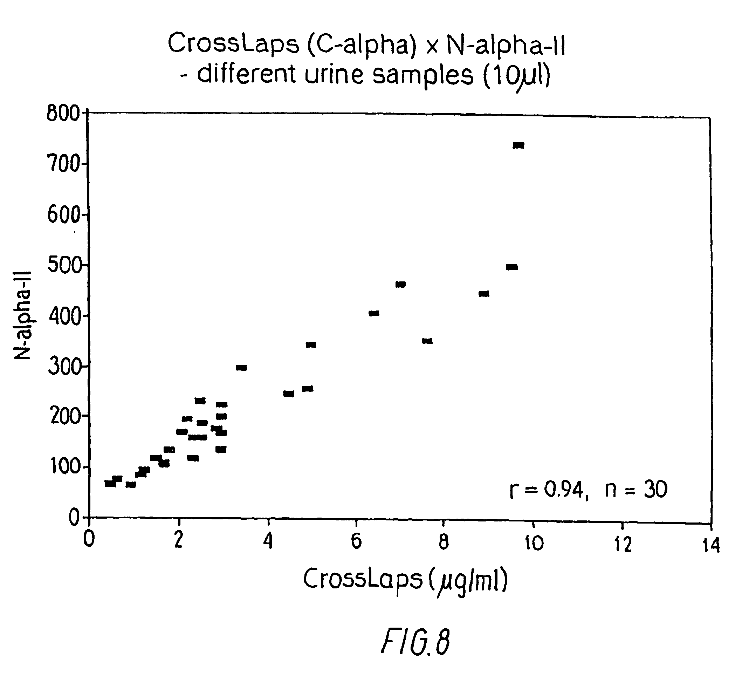

Figure 8 shows the detection by MAbA7 ELISA of the increase in bone resorption associated with the menopause,

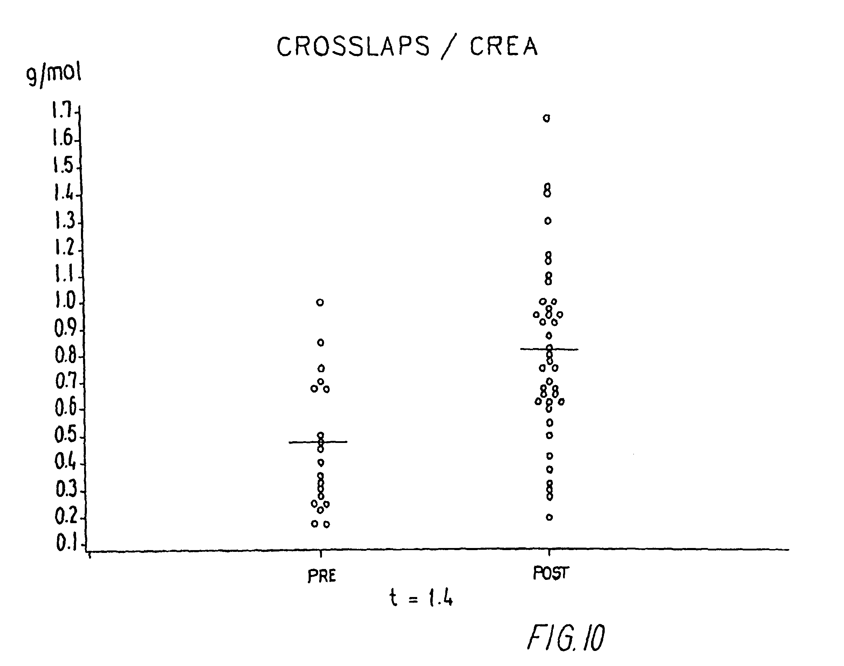

Figure 10 shows the detection by CrossLaps ELISA of the increase in bone resorption associated with the menopause,

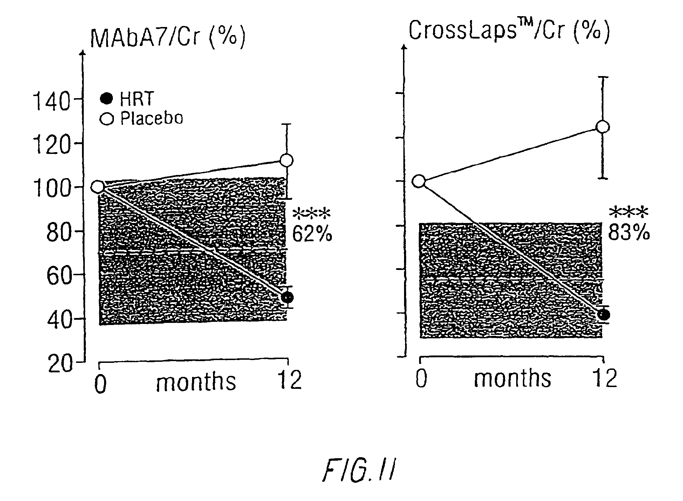

Figure 11 shows the change in biochemical markers during hormone replacement therapy as determined by CrossLaps ELISA and MAbA7 ELISA,

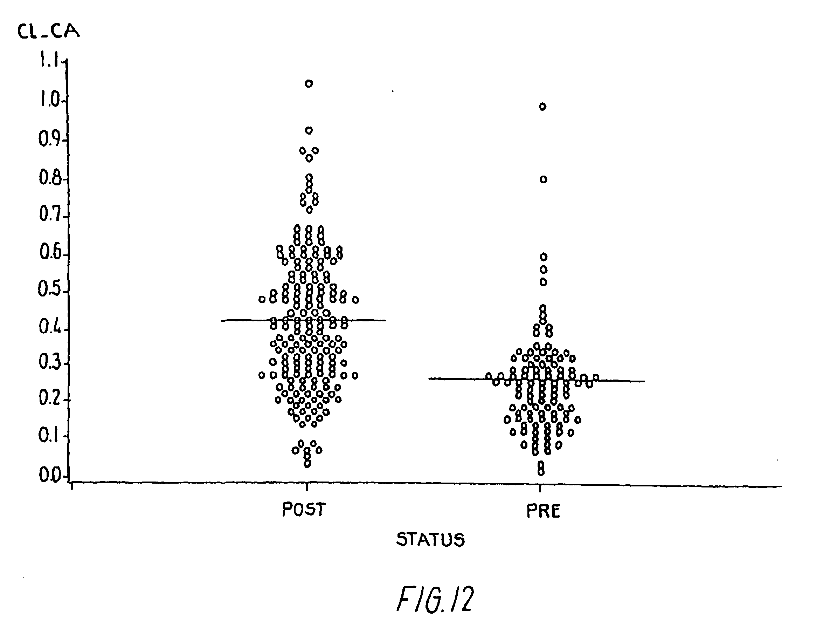

Figure 12 shows the individual values of the measurement in CrossLaps of urine samples from normal, age-matched women,

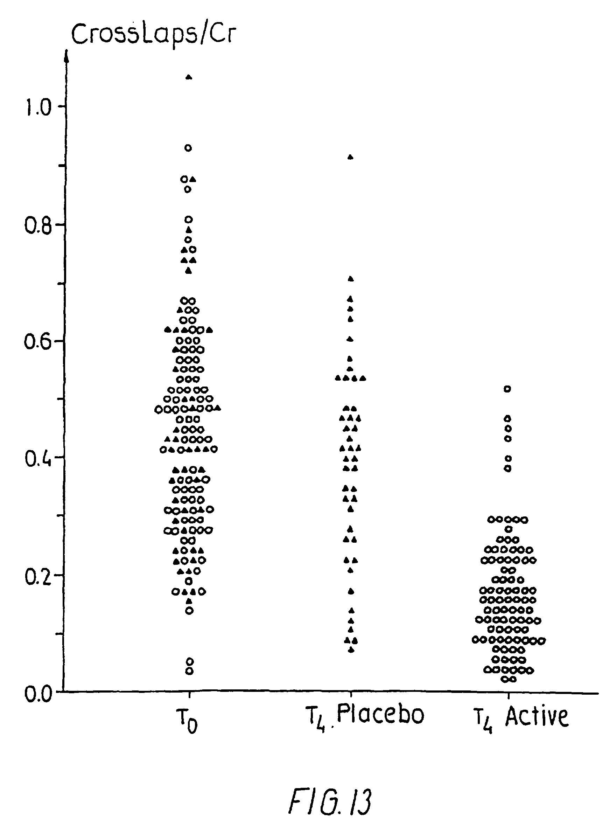

Figure 13 shows the individual values of the measurement of urine samples from women on a hormone replacement therapy and placebo in the CrossLaps immunoassay,

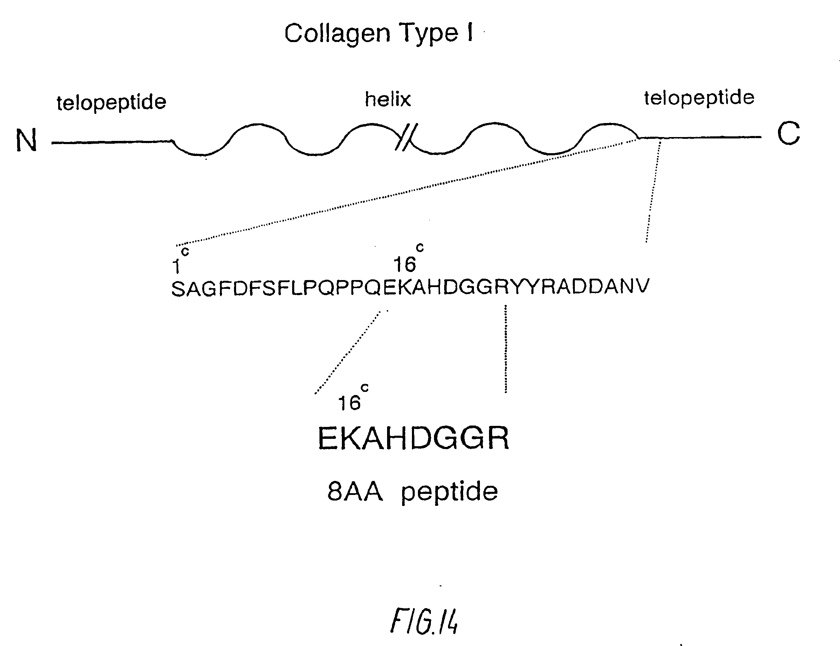

Figure 14 is a schematic presentation of the location of the 8AA peptide selected for the CrossLaps immunoassay,

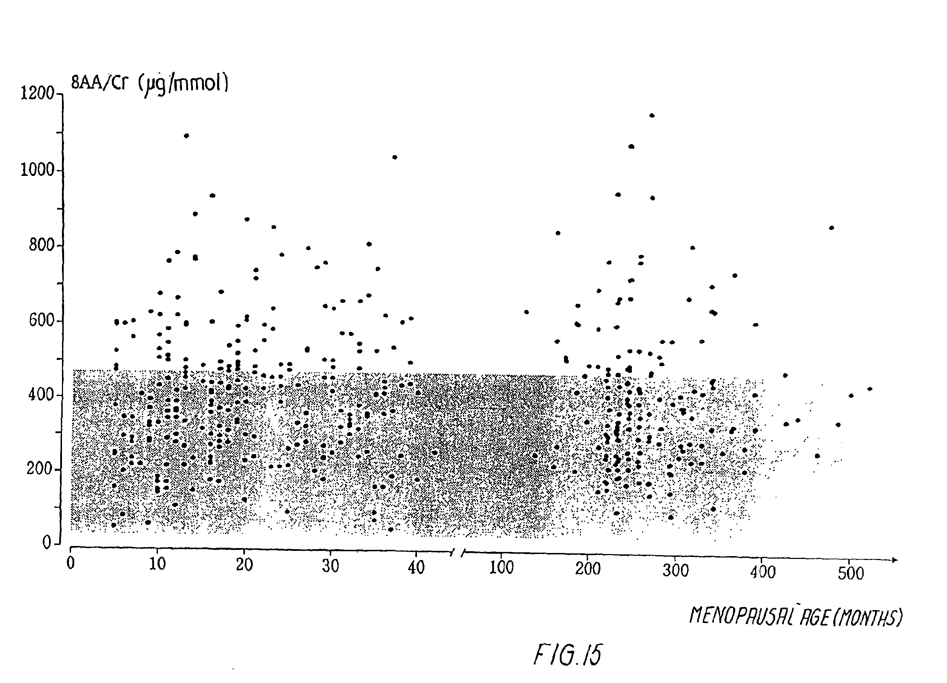

Figure 15 shows individual 8AA values from early postmenopausal women (n=245) and late menopausal women. The hatched area shows the mean values ± 2 SD from a group of premenopausal women (n=102),

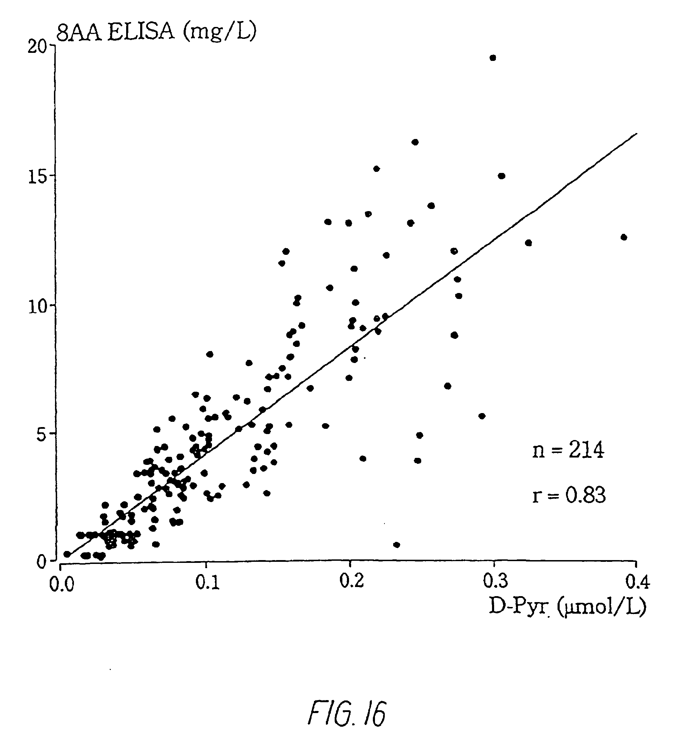

Figure 16 shows the correlation between D-Pyr and 8AA ELISA. On 214 healthy women aged 30.0-65.0, values obtained in the 8AA ELISA were compared to D-Pyr (HPLC method),

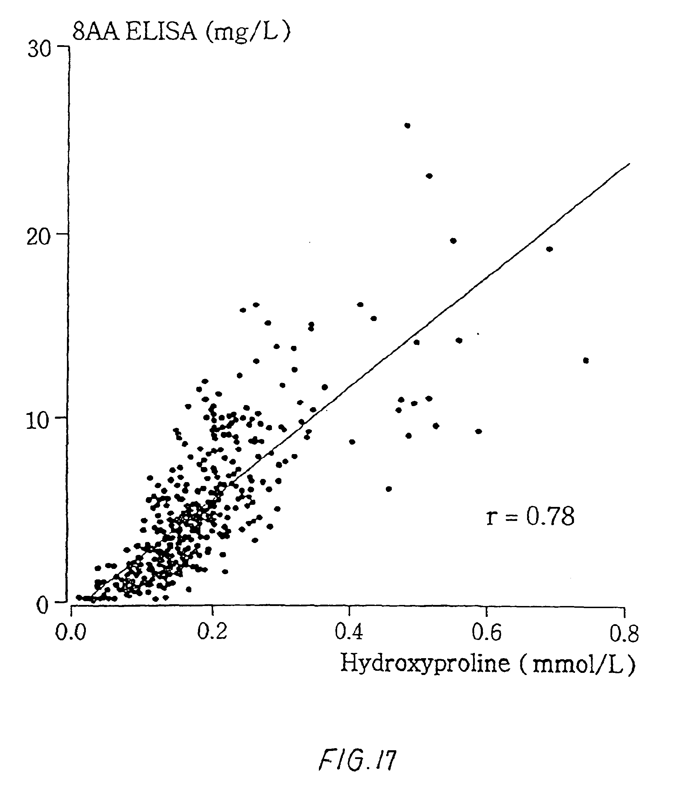

Figure 17 shows the correlation between Hpr and the 8AA ELISA. On 421 healthy women aged 30.0-54.0 years, values obtained in the 8AA ELISA were compared to Hpr;

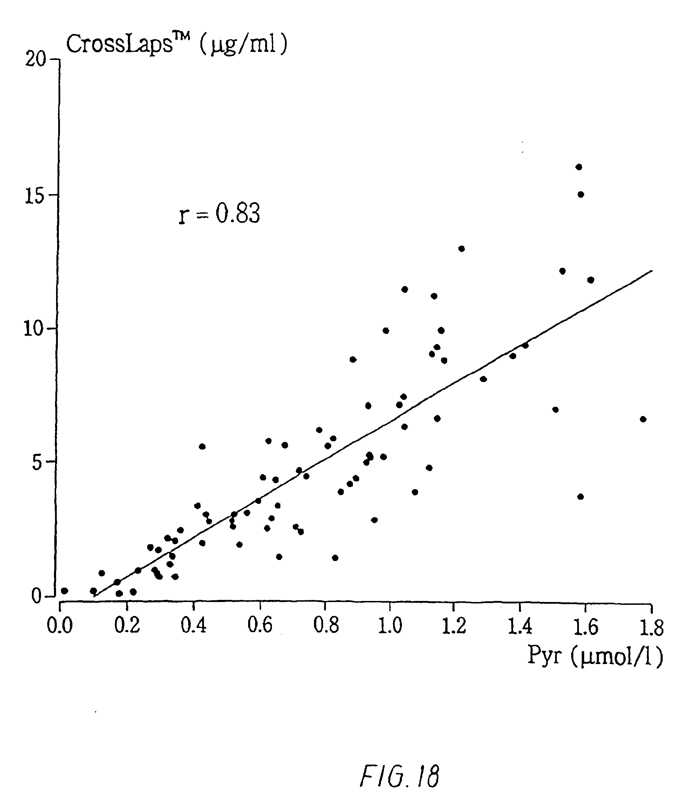

Figure 18 shows the correlation between values obtained in the CrossLaps™ ELISA and Pyridinoline on HPLC (n=81) ;

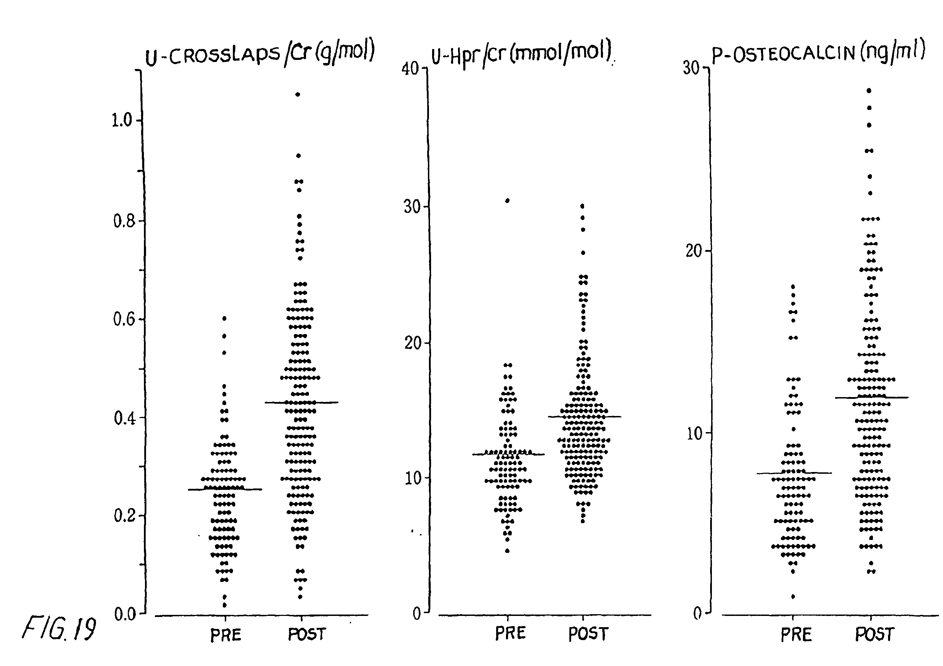

Figure 19 indicates CrossLaps™, Hpr and osteocalcin in pre- (n-104) and postmenopausal (n-180) women. T-scores are given at the bottom panel;

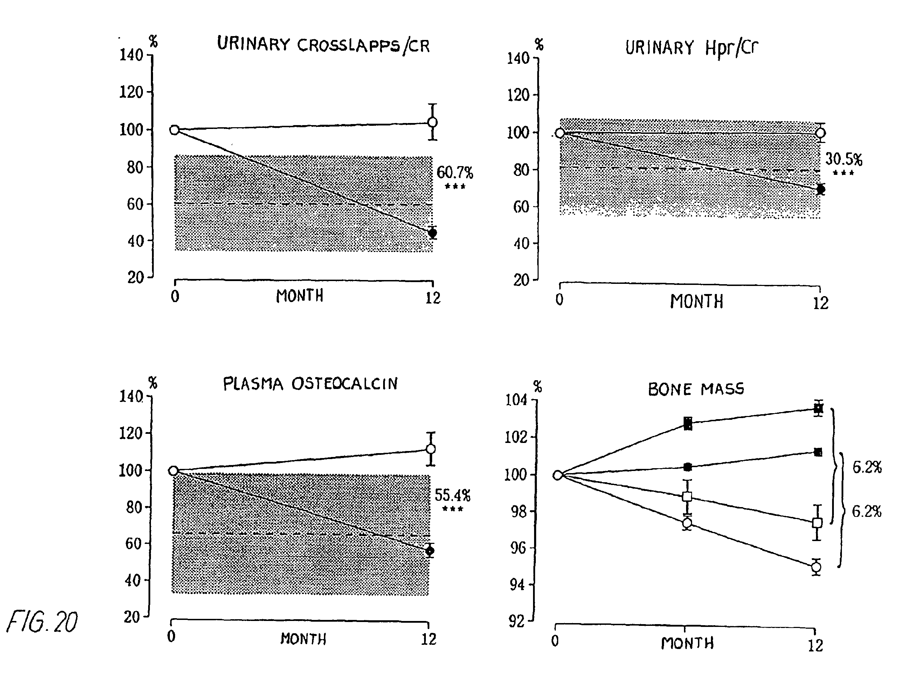

Figure 20 shows the mean changes of the biochemical parameters and forearm (o) and spinal (.) bone mass after 12 months' hormone replacement (n-80) (.) or placebo (n=35) (o) therapy expressed as a per cent of baseline values. The hatched areas represent the variation in the premenopausal women; and

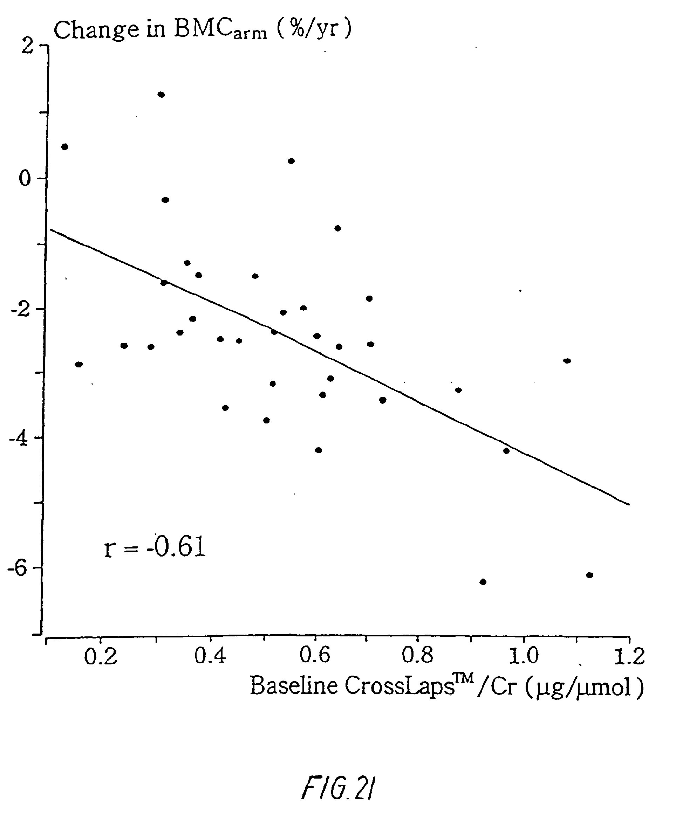

Figure 21 shows the correlation between baseline values of CrossLaps™/Cr and the rate of loss (% per year) determined by 9 forearm bone mass measurements over 24 months (n=35).

In a preferred embodiment of the method according to the invention, the assaying of Type I collagen fragments in urine is performed by an inhibition ELISA (enzyme linked immunosorbent assay) by metering off a sample of urine and contacting the sample with a synthetic peptide having a sequence derived from collagen and with an antibody, which is immunoreactive with the synthetic peptide. The synthetic peptide is immobilised on a solid support. The antibody is raised against the synthetic peptide.

The combined reagents and sample are incubated, and a peroxidase-conjugated (revealing) antibody is added. After another incubation, a peroxidase substrate solution is added. Following short final incubation, the enzyme reaction is stopped, and the absorbance is measured at 450 nm and compared with a standard curve obtained with standard solutions by the same procedure.

Synthetic peptides are used for the preparation of standards. The concentration of synthetic peptide in a stock solution of the relevant synthetic peptide is determined by quantitative amino acid determination. A two-fold dilution of the stock solution is prepared and subsequently used for the construction of the standard curve in the inhibition ELISA.

### Preparation of synthetic peptides

The preparation of synthetic peptides may be performed according to procedures well known in the art, e.g. by solid-phase peptide synthesis techniques commonly described as "Merrifield synthesis". Also classical solution phase techniques may be used. Sequences of interest include potential sites for cross-linking (see for example Kühn, K., in Immunochemistry of the extracellular matrix, 1:1-29 (1982), Eyre, D.R., Ann.Rev. Biochem. 53:717-48 (1984), or US Patent No. 5,140,103). Examples of such peptides sequences are given in Table 1 below.

Regarding the synthetic peptides, it is possible to omit (or add) one or more amino acid residues from (or to) the sequences given above without substantial loss of the ability to (a) raise antibodies recognising the corresponding native collagen fragment or (b) inhibit the bindings of such antibodies to the native fragment. It is possible to use longer collagen fragments and/or chimeric peptides to raise the antibodies and, in principle, it is not necessary to use the same peptide as the immunogen and the competitor in the assay.

### Preparation of antibodies

The methods for preparation of both monclonal and polyclonal antibodies are well known in the art. For example, see Campbell, A.M., Laboratory Techniques in Biochemistry and Molecular Biology, Vol. 13 (1986). It is possible to produce antibodies to synthetic peptides by immunisation. However, because of the relatively small molecular weight of these compounds it is preferred that the hapten be conjugated to a carrier molecule. Suitable carrier molecules include, but are not limited to, bovine serum albumin, thyroglobulin, ovalbumin, tetanus toxoid, and keyhold limpet hemocyanin. The preferred carrier is bovine serum albumin. To present the hapten in its most immunogenic form to the antibody producing cells of the immunised animal a number of alternative coupling protocols can be used. Suitable procedures include, but are not limited to, glutaraldehyde, carbodiimide, and periodate. Preferred binding agents are glutaraldehyde and carbodiimide.

The preparation of antibodies is carried out by conventional techniques including immunization with collagen fragments or synthetic peptides conjugated to a carrier. To improve the immunogenicity it is preferred that the immunogen be mixed with an adjuvant before injection. Examples of adjuvants include, but are not limited to, aluminum hydroxide, Freund's adjuvant, and immune-stimulating complexes (ISCOMs). ISCOMs can be made according to the method described by Morein, B. et al., Nature 308:457-460 (1984).

Either monoclonal or polyclonal antibodies to the hapten carrier molecule can be produced. For the production of monoclonal antibodies it is preferred that mice are immunized. Spleen cells from the immunized mouse are harvested, homogenized, and thereafter fused with cancer cells in the presence of polyethylene glycol to produce a cell hybrid which produces monoclonal antibodies specific for peptide fragments derived from collagen. Suitable cancer cells include, but are not limited to, myeloma, hepatoma, carcinoma, and sarcoma cells. Detailed descriptions of the production of monoclonal antibodies are provided in Goding, J.W., in Monoclonal Antibodies: Principles and Practice, (1986). A preferred preliminary screening protocol comprises the use of synthetic peptides conjugated to a carrier and coated onto the solid surface of a microtitre plate.

For the preparation of polyclonal antibodies, which are reactive with peptide fragments derived from collagen, different animal species can be immunized. Suitable species include, but are not limited to, chicken, rabbit and goat. Chicken and rabbit are preferred.

Antibody fragments are prepared by methods known in the art (see E. Ishikawa. Journal of Immunoassay 3:209-327 (1983)).

### Conduct of Immunoassays

Accordingly, by utilization of an immunoassay with the antibodies prepared as above it is possible to assay a biological fluid sample without prior fractionation or hydrolysis. The specificity for the desired collagen in the biological fluid is supplied by the antibody in combination with the use of a synthetic peptide (against which the antibody was raised or in any event with which the antibody is immunochemically reactive) in the assay construction.

As an alternative the immunoassay may be performed using a monoclonal antibody. The basic idea of this assay design is to shift the specificity of the assay from the antigen (synthetic peptide to collagen) to the antibody (from rabbit antiserum to monoclonal antibody). Using this construction the assay does not need to make use of a synthetic peptide. This version of the immunoassay is performed by incubating the patient sample or a standard solution with a peroxidase-conjugated antibody solution in a microtiter plate pre-coated with purified collagenase-treated collagen. After washing, the wells of the plate are incubated in the dark with a substrate solution. The colour reaction is stopped by the addition of a stopping solution, and finally the absorbance is measured.

The immunoassays themselves are conducted using any procedure selected from the variety of standard assay protocols generally known in the art. As it is generally understood, the assay is constructed so as to rely on the interaction between the specific immunological binding partner and the desired analyte for specificity and to utilize some means to detect the complex formed by the analyte and the immunological binding partner. The immunological binding partner may be complexed to a solid support and used as a capture immunological binding partner for the analyte. This protocol may be run in a direct form, wherein the formation of analyte/immuno-logical binding partner complex is detected, e.g. by a fluorescent, radioactive or enzymatic label, or it may be run in a competitive format wherein a labelled standard competes with the analyte for the immunological binding partner. The format may also be constructed as an agglutination assay or the complex may be precipitated by addition of a suitable precipitant to the reaction mixture. The specific design of the immunoassay protocol is open to a wide variety of choice, and the number of clinical assay devices and protocols available in the art is multitudinous. For a variety of such protocols, see U.S. Pat. No. 5,001,225.

The antibodies and revealing reagents for the conduct of an immunoassay using standard detection protocols, for example radioisotope labelling, fluorescent labelling or ELISA, either in a direct or competitive format, may conveniently be supplied as kits which include the necessary components and instructions for the assay. In one embodiment of the invention such a kit includes a microtiter plate coated with a relevant synthetic peptide, standard solutions for preparation of standard curve, a urine control for quality testing of the analytical run, rabbit antibodies reactive with the above-mentioned synthetic peptide, anti-rabbit immunoglobulins conjugated to peroxidase, a substrate solution, a stopping solution, a washing buffer and an instruction manual.

Since immunoassays can be constructed using antibodies and specific synthetic peptides, the ratios of the corresponding collagen fragment sequences in an appropriate biological fluid can be determined as well as their individual levels and their total. Thus, the assay can be designed to include antibodies which will result in determination of several native peptide sequences or determination of a single peptide sequence, or any desired combination thereof.

In addition to the use of the herein specified peptides as indicators of bone resorption, bone metabolic balance is advantageously determined by the substantially simultaneous determination of a marker of the formation of bone in the same or other appropriate biological fluid from the same individual. "Substantially simultaneous" means the same day, preferably within 4 hours. For example such markers include osteocalcin (also known as bone GLA protein of BGP), procollagen Type I, bone alkaline phosphatase and total alkaline phosphatase. Suitable methods for the determination of these markers can be found, for example, in Delmas, P.D., et al., J. Bone Min. Res. (1986) 1:333-337.

The assay of the present invention allows one to assess an abnormal condition of a subject by indicating, for example, excessive bone resorption. This may show the presence of an osteoporotic condition or the metastatic progress of a malignancy. Other conditions characterised by excessive bone resorption include Paget's disease and hyperparathyroidism. Further the assays can be used as a measure of toxicity, since the administration of toxic substances often results in tissue degradation.

Thus the assays may be applied in any situation wherein the metabolic condition of Type I collagen tissues can be used as an index of the condition, treatment, or effect of substances directly administered to the subject or to which the subject is exposed in the environment.

The following examples are intended to illustrate the principles used in the invention, but not to limit the invention. Some of the Example below use sequences other than as claimed in this invention, but they still illustrate the principles involved.

### EXAMPLE 1: IMMUNOASSAYS FOR SPECIFIC PEPTIDE SEQUENCES IN URINE

Three peptides (α1(I)C1, α1(I)N1, and *α*2(I)N1) (see Table 1 below) prepared by solid-phase techniques are used for the preparation of immunogens. *α* 2(I)N1 is in accordance with the present invention. For immunisation, the peptides are covalently attached to bovine serum albumin using glutaraldehyde reagents and methods well known in the art. Both monoclonal and polyclonal antibodies are raised against the peptides. For production of monoclonal antibodies, Balb/c mice are immunised with peptide-BSA conjugates, and hybridoma cell lines are prepared using standard techniques after fusion of cells from the spleen or lymph nodes with Ag8 myeloma cells. Polyclonal antibodies are raised in rabbits and chickens. Screening of both antisera and hybridoma cell media were performed by ELISA using microtiter plates coated with the appropriate peptide-gelatin conjugate prepared using carbodiimide reagents and methods well known in the art.

**TABLE 1**

| | N | C |
|---|---|---|
| α1 (I) N-term. | Asp-Glu-Lys-Ser-Thr-Gly-Gly | (α1(I)N1) |
| α1 (I) C-term. | Glu-Lys-Ala-His-Asp-Gly-Gly-Arg | (α1(I)C1) |
| α2 (I) N-term. | Gln-Tyr-Asp-Gly-Lys-Gly-Val-Gly | (α2(I)N1) |

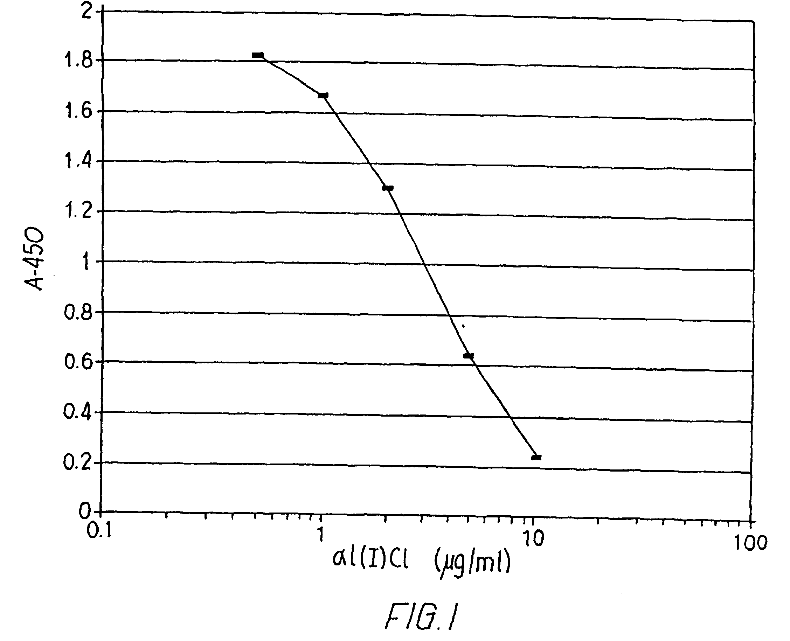

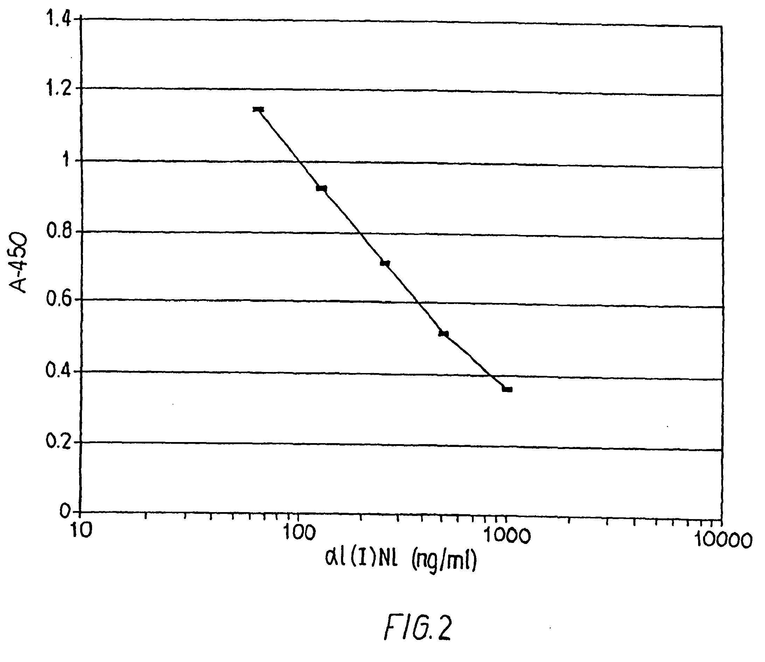

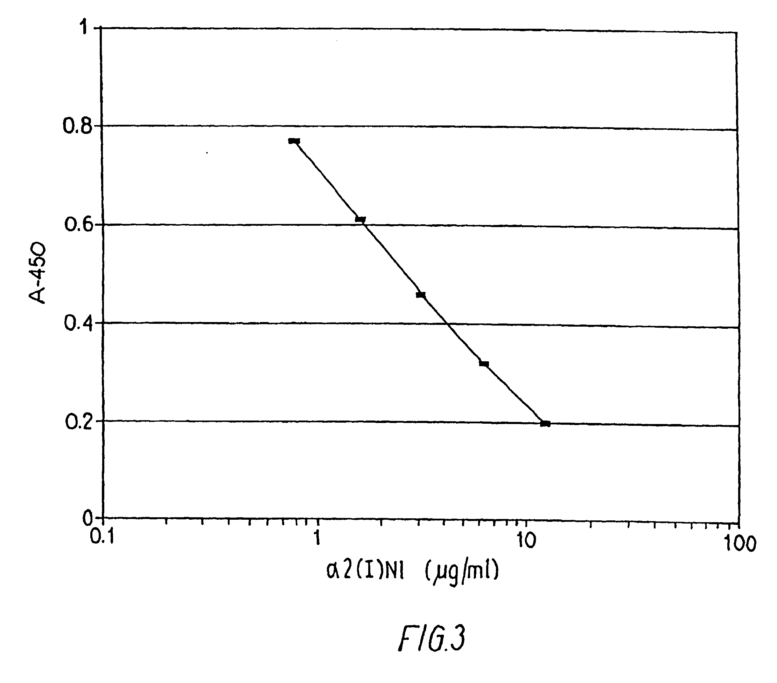

Assays for three of the peptide sequences (α1(I)C1), α1(I)N1, and α2(I)N1) in urine are performed by an inhibition ELISA as follows:
Urine samples (10 or 25 *µ*l) possibly containing collagen fragments or solutions containing 0.05-15 *µ*g peptide/ml as reference standards, respectively, are added to 75 *µ*l of immunological binding partners for the peptides diluted 1:5,000 - 1:20,000 in phosphate buffered saline containing 0.1% Tween-20 detergent (PBS-T) and including 0.1% (w/v) of BSA. Each sample is prepared in duplicate in flat-bottomed, 96-well microtiter plates previously coated with gelatine conjugate containing the appropriate peptide. After 60 minutes, the plates are washed with PBS-T (3 times) and the bound antibodies are detected by standard techniques with a horse radish peroxidase labelled antibody prepared against the species of the primary antibody. Peroxidase substrate is added and the colour development is measured at 450 nm in an automated microtiter plate reader after stopping the enzyme reaction using 1 M H₃PO₄. Samples containing the analyte decrease the binding of primary antibody to the immunobilised peptide in the plate and thus have a reduced colour concentration. The amount of analyte in the sample is quantified with reference to previously established curves from standards included on each plate computed using log-lin plots. Figures 1, 2 and 3 show typical standard curves for the α1(I)C1 (CrossLaps) immunoassay (Fig. 1), α1(I)N1 immunoassay (Fig. 2), and α2(I)N1 immunoassay (Fig. 3).

### EXAMPLE 2: CORRELATION TO PYRIDINOLIN DETERMINATION ON HPLC

On a number of unselected urine samples the concentration of total pyridinolin (HPLC method, see for example Uebelhart, D., Bone and Mineral, 8:87-96 (1990)) was measured. Values obtained in this HPLC system were correlated to the values obtained in two immunoassays (CrossLaps and an assay based on α1(I)N1 peptide).

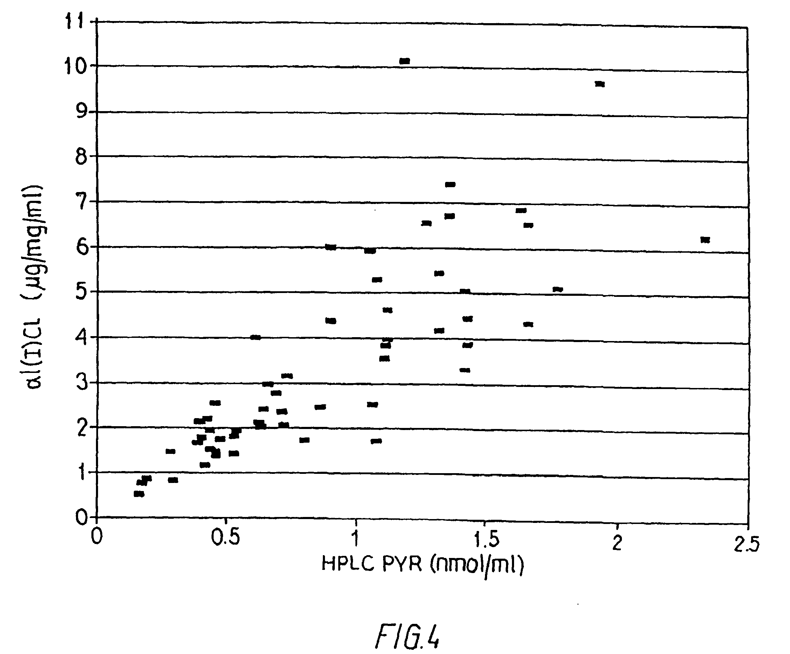

Figure 4 shows the correlation between total pyridinolin (HPLC) and the CrossLaps immunoassay (n=59). The correlation calculated by linear regression analysis is r=0.80.

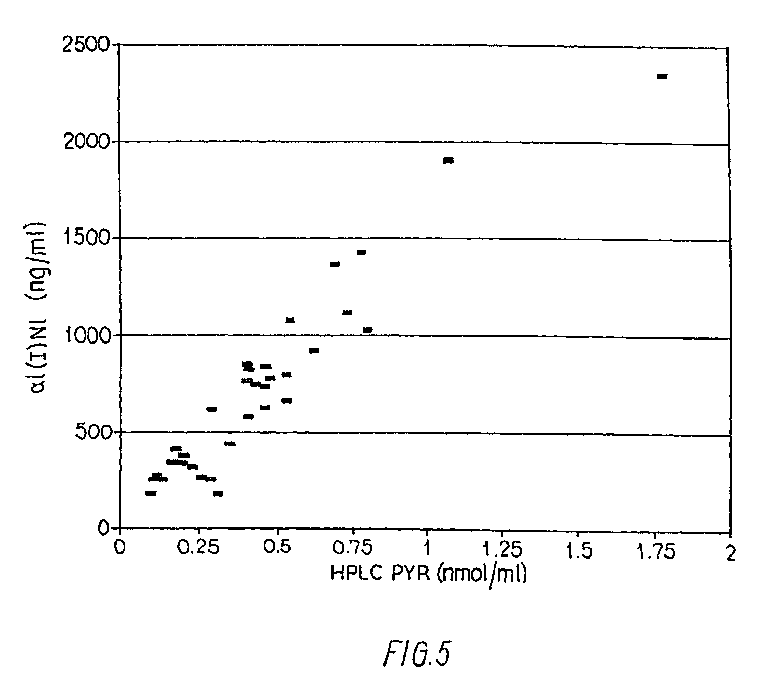

Figure 5 shows the correlation between total pyridinolin (HPLC) and α1(I)N1 immunoassay (n=36). The correlation calculated by linear regression analysis is r=0.95.

### EXAMPLE 3: ASSAY USING A MONOCLONAL ANTIBODY FOR DETECTION OF DEGRADATION PRODUCTS OF TYPE I COLLAGEN

Monoclonal antibodies were developed by immunization of mice with α1(I)C1 synthetic peptide conjugated to an appropriate carrier protein. Cell fusion, cloning and propagation of hybridomes were performed according to standard procedures. Screening procedures included testing of reactivity to α1(I)C1 synthetic peptide immobilized in microtiter plates.

The specificity of the antibodies was tested by inhibition studies using different overlapping sequences from the C-telopeptide of type I collagen.

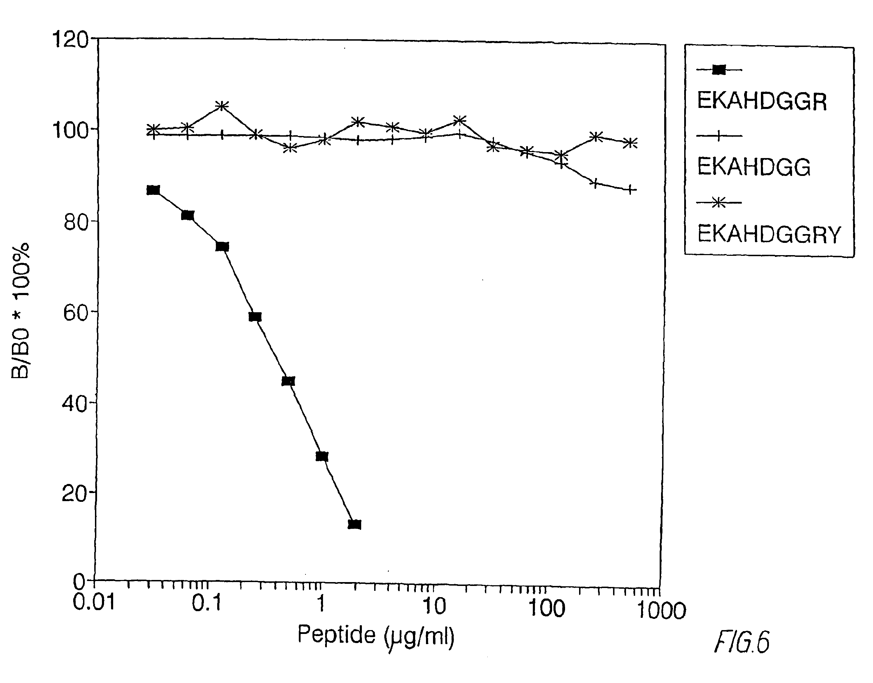

The specificity of one such antibody, MAbA7, is demonstrated in Figure 6.

An assay using the antibody MAbA7 was developed. In brief, the synthetic peptide α1(I)C1 was conjugated to bovine serum albumin using glutaraldehyde, and the conjugate was used for coating of microtiter plates. Alternative material can also be used, the essential feature being exposure of the sequence EKAHDGGR cleaved after arginine (R).

One such alternative is type I collagen treated with bacterial collagenase. Another alternative could be expression of EKAHDGGR sequence in a recombinant peptide, the essential feature again being exposure of the carboxy-terminal end of the EKAHDGGR sequence (i.e. the arginine residue).

Following coating, the wells of the microtiter plates are incubated with 15 µl of urine and 100 µl of MAbA7 conjugated to horseradish peroxidase.

After one hour the plates are washed and substrate (e.g. TMB) is added.

The epitope specificity of MAbA7 appears from Fig. 6. The reactivity of MAbA7 to immobilized EKAHDGGR in microtiter plates was inhibited by co-incubation with different synthetic peptides. The results indicate that cleavage after arginine is essential for detection in MAbA7 ELISA.

### EXAMPLE 4: CORRELATION OF CROSSLAPS ELISA TO ELISA ASSAYS BASED ON OTHER PEPTIDE FRAGMENTS AND ANTIBODIES

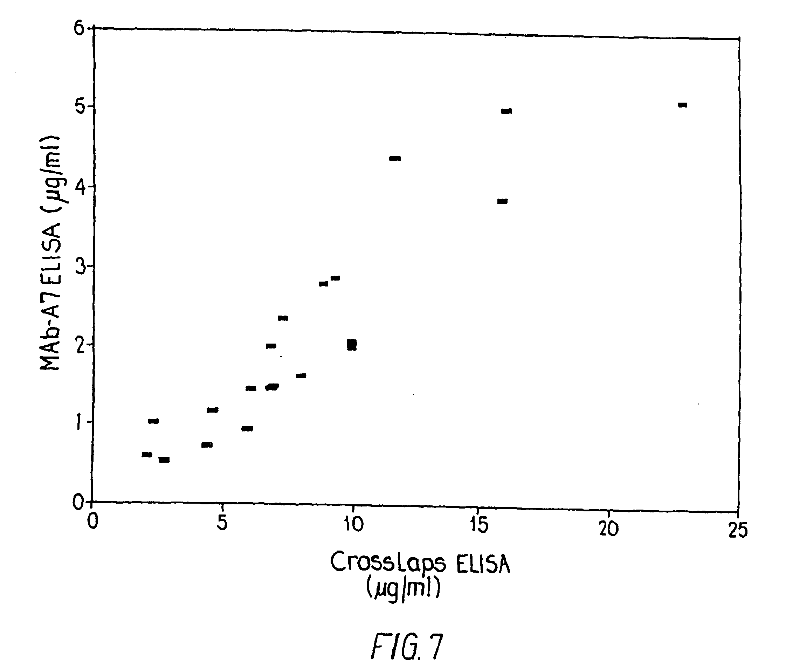

The correlation between the CrossLaps ELISA and an MAbA7 ELISA on urine from 20 postmenopausal women is shown in Fig. 7. The MAbA7 ELISA is a competitive assay wherein the synthetic 8AA peptide (EKAHDGGR) immobilized in a microtiter tray competes with collagen fragments in the patient sample about the binding to the monoclonal antibody (MabA7).

The results shows a high concentration which indicates that the two assays reflect the same or related metabolic processes.

Similarly the correlation between CrossLaps and Nα2 ELISA is shown in Fig. 8 Nα2 is a competitive assay wherein the Nα2(I)N1 peptide fragment (QYDGKGVG) from the N-telo-peptide in the α2-chain in Type I collagen competes with collagen fragments in the patient sample about the binding to an anti-QYDGKGVG antiserum raised analogously with the description above. The results show a high correlation which indicates that the two assays reflect the same or related metabolic processes.

### EXAMPLE 5: INVESTIGATION OF THE CROSSLAPS ELISA

The synthetic peptide Glu-Lys-Ala-His-Asp-Gly-Gly-Arg (or EKAHDGGR) has been incorporated in an Enzyme Linked Immunosorbent Assay (ELISA) denominated "CrossLaps™", as mentioned above. The CrossLaps ELISA has been investigated by a group of French specialists (Garnero et al) and the results will be published in J.Clin.Indocrinol. Metab. 79, No.3 (1994).

A summary of the investigations is provided below.

The CrossLaps ELISA is based on an immobilised synthetic peptide with an amino acid sequence of eight amino acids (8AA) specific for a part of the C-telopeptide of the α1-chain of Type I collagen (Glu-Lys-Ala-His-Asp-Gly-Gly-Arg, CrossLaps antigen). During incubation with an antibody raised to this sequence, a competition takes place between the immobilised peptide and the breakdown products of the α1-chain of type I collagen in urine.

Briefly, a 25 µL urine sample or standard is added to each well of a CrossLaps antigen-coated microplate, followed by 75 µL of an anti-CrossLaps antiserum. The plates are incubated for 1 h at room temperature under agitation and washed five times with a washing buffer. A goat antirabbit immunoglobulin G horseradish peroxidase conjugate (100 µL) is added to each well. After incubation for 1 h at room temperature, plates are washed five times as before. The enzyme substrate (100 µL/well) is added, and after 15 min of incubation in the dark, the reaction is stopped by adding 100 µL phosphoric acid (1 mol/L). The optical density at 450 nm is measured with a microplate reader. Duplicate measurements are performed for each urine sample, and the data are expressed as micrograms per mmol urinary creatinine (Cr), measured by a standard colorimetric technique.

In some samples, the total excretion of Pyr and D-Pyr was also measured on a hydrolyzed sample by HPLC, and urinary hydroxyproline was determined by a spectrophotometric method.

The CrossLaps ELISA was used to measure the urinary excretion of type I collagen peptides released during bone matrix degradation in a sample of healthy adults comprising 146 women and 60 men, aged 31-89 years, and in patients with metabolic bone disease. The intra- and interassay coefficients of variation were less than 10% and 13%, respectively. The recovery of CrossLaps antigen from urine samples ranged from 92-115%, and the ELISA was linear for serial sample dilutions. The CrossLaps assay does not cross-react with either free pyridinoline (Pyr) or free deoxypyridinoline (D-Pyr). CrossLaps measured by ELISA and the total excretion of Pyr measured by high performance liquid chromatography were highly correlated in normal women (n=91; r=0.73; P<0.001). Urinary CrossLaps excretion increased with age in women, but not in men. In women, the menopause was reflected by a mean 141% increase in CrossLaps excretion of bone alkaline phosphatase (+48%) and osteocalcin [+41%; from an average 217 to 524 µg/mmol creatinine (Cr)] that was higher than the mean increase in total D-Pyr (+91%) and total Pyr (+47%) measured by HPLC. Urinary CrossLaps excretion was increased from control values in Paget's disease (n=32; mean, 1810 ± 2300 µg/mmol Cr; P<0.001), and in patients with primary hyperparathyroidism (n=10; mean, 780 ± 380 µg/mmol Cr; P<0.001), and in patients with hyperthyroidism (n=27; mean, 1280 ± 970 *µ*g/mmol Cr; P<0.001), with Z-scores (number of SD from the mean of sex- and age-matched controls) of 4.4 ± 6.6, 1.5 ± 1.2, and 6.7 ±6.5, respectively. In patients with Paget's disease, CrossLaps values were highly correlated with urinary hydroxyproline levels (r=0.91; P<0.001), and the decrease in urinary CrossLaps excretion was greater than that in urinary hydroxyproline (-71% vs. - 17%; P<0.001) after 3 days of intranenous treatment with the bisphosphonate pamidronate. In patients with hyperthyroidism, CrossLaps excretion was elevated above the normal range in most patients (78%) and returned to normal within 1 month of treatment for hyperthyroidism.

Based on these observations Garnero et al. conclude that this new convenient assay represents a sensitive and specific index of the bone resorption rate, and that it should be useful for the clinical investigation and therapeutic monitoring of patients with osteoporosis and other metabolic bone diseases.

### EXAMPLE 6: INVESTIGATION OF THE MAbA7 ELISA

As mentioned previously the assay construction based on the monoclonal antibody MAbA7 does not need to use a synthetic peptide. This assay version is carried out in three steps as follows:

In the first step the wells of a microtiter plate pre-coated with purified collagenase-treated collagen (CTC) are incubated for 1 hour at room temperature with 15 µl standard solution or urine sample and 100 µl of peroxidase-conjugated monoclonal antibody solution.

After washing in the second step the wells are incubated in the dark for 15 minutes at room temperature with 100 µl substrate solution. Finally, in the third step, the colour reaction is stopped by the addition of 100 µl stopping solution. The absorbance at 450 nm is measured within 2 hours.

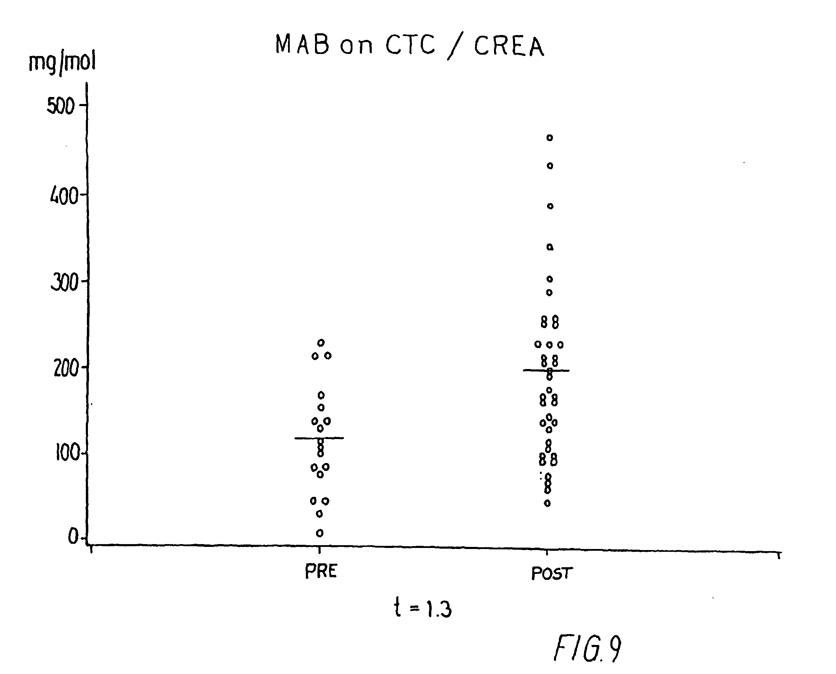

Figure 9 shows the detection of MAbA7 ELISA of the increase in bone resorption associated with the menopause. Urine samples from 18 premenopausal and 38 postmenopausal women were tested. The t-score (difference in mean values expressed as the number of standard deviations of the premenopausal women) indicates a relatively fine separation between the two populations.

Figure 10 shows the similar detection of the increase in bone resorption associated with the menopause carried out by CrossLaps ELISA. The urine samples are identical to those mentioned in Figure 8.

The change in biochemical markers during hormone replacement therapy (HRT) appears from Figure 11. Urine samples from women receiving oestrogen (solid circles) or placebo (open circles) were tested at baseline and after 12 months of therapy. Values after 12 months are expressed as percentages of the baseline values. The results indicate that CrossLaps ELISA and the MAbA7 ELISA (using CTC or the synthetic peptide EKAHDGGR as coating) are equally sensitive to anti-resorptive treatment.

### EXAMPLE 7: CLINICAL RESULTS

The immunoassay procedure (using the α1(I)C1 peptide, i.e. the CrossLaps ELISA) set forth in Example 1 was applied to urine samples from different individuals, and the amount of analyte was quantitated. The values obtained were related to the level of urinary creatinine in the urine sample as is commonly done for urine assays. The values obtained for normal, age-matched women (premenopausal and postmenopausal) are shown in Table 2.

**TABLE 2**

| Measurement of α1(I)C1 peptide in urine samples from normal, age-matched women (premenopausal and postmenopausal) | |
|---|---|
| Group | α1(I)C1 peptide (mg/mmol creatinine) |
| Premenopausal (n=104) | 0.263±0.143 |
| Postmenopausal (n=180) | 0.426±0.190 |

For individual values see Fig. 12.

The difference between the premenopausal and the postmenopausal values is highly significant (P<0.0001).

The z-score of a quantitative test procedure shows the ability of the procedure to distinguish between two populations. Table 3 below shows the z-score of the CrossLaps immunoassay and the state of the art measurement of pyridinolin on HPLC when applied to the same set of urine samples from normal, age-matched premenopausal (n=104) and postmenopausal women (n=180).

**TABLE 3**

| Z-scores obtained for two different test procedures (total pyridinolin (HPLC) and the CrossLaps immunoassay) when applied to urine samples from normal, age-matched women (premenopausal and postmenopausal) | |
|---|---|
| Test procedure | Z-score |
| α1(I)C1 immunoassay (CrossLaps) | 1.14 |
| Total pyridinolin (HPLC) | 1.25 |

As can be seen from Table 3, the discriminatory powers of the two methods are approximately alike.

For an assay to be used as an index of bone resorption it is very important to be able to measure the impact of a hormone replacement therapy (HRT). Table 4 shows the results from the CrossLaps ELISA in such a study.

**TABLE 4**

| Measurement of urine samples from women on a hormone replacement therapy and placebo in the CrossLaps ELISA | | |
|---|---|---|
| Group | α1(I)C1 peptide t=0 | (mg/mmol creatinine) t=12 months |
| HRT (n=92) | 0.457+0.176 | 0.176+0.103 |
| Placebo (n=45) | 0.459±0.209 | 0.402±0.187 |

For individual values see Fig. 13.

A highly significant drop in the group receiving HRT is seen after 12 months (P<0.001).

### EXAMPLE 8: EVALUATION OF THE CROSSLAPS ELISA IMMUNOASSAY FOR QUANTITATION OF TYPE I COLLAGEN DEGRADATION PRODUCTS IN URINE

Pyridinoline (Pyr) and deoxypyridinoline (D-Pyr) are crosslinks between mature collagen molecules and are primarily found in bone and cartilage whereas they are absent in skin. D-Pyr is more specific for bone than Pyr but neither are absolutely bone specific. The determination of the total excretion of Pyr and D-Pyr, however, can be used as an index of bone resorption. Pyr and D-Pyr are usually measured fluorometrically in hydrolysed urine after separation using HPLC, which is a time consuming and complicated method not suitable for routine use.

This example describes an assay, i.e. CrossLaps ELISA which measures in urine a type I collagen specific peptide sequence independently of the Pyr and D-Pyr structures. As more than 90 % of the organic matrix of bone consists of type I collagen the peptide sequence measured in this assay is a potential marker of bone resorption.

For the establishment of a reference value for the assay urine samples from 102 healthy premenopausal women, aged 30-51 years (mean± 1SD; 39.3± 5.56) were assayed. All women had a history of regular vaginal bleeding and none were taking any medication known to influence the calcium metabolism. Likewise, samples from early postmenopausal women (n=245), aged 45-57 years (mean± 1 SD; 51.1± 2.38) were assayed. All these women had a history of a natural menopause (mean menopause age 20.1±9.90 months). Furthermore, samples from late postmenopausal women (n=165), aged 68-72 years (mean± 1 SD; 70.0± 1.19) were assayed. All these women also had a history of a natural menopause (mean menopause age 268 ± 68.2 months). None of these women were taking any medication known to influence the calcium metabolism.

For the comparison of the assay tested and D-Pyr (HLPC method), urine samples from 214 healthy women aged 30.0-65.0 years were assayed. For the comparison of the assay and Hpr, samples from another population of healthy women aged 30.0-54.0 years were assayed (n=421).

The experiment was carried out essentially as outlined in example 5 with the CrossLaps ELISA based on the 8AA specific synthetic peptide. In brief, 25 µL Standard or unknown sample is pipetted in duplicates into the appropriate wells in the pre-coated ELISA plate. Then 75 µL Antibody Solution (rabbit antibodies to 8AA) is added to each well, the plate is covered with sealing tape and incubated at room temperature for 60 min on a shaking device. All the following procedures were also carried out at room temperature. After incubation the plates were washed three times with diluted Washing Buffer.

Peroxidase conjugated Antibody (HRP- conjugated goat antibodies to rabbit IgG, 100 µL/well) was added and the sealed wells were incubated 60 min on a shaking device. Following another washing procedure, 100 µL of TMB Substrate Solution was added to all wells which were sealed and incubated for 15 min. The enzyme reaction was stopped after 15 min by addition of 100 µL Stopping Solution. The optical density was read in an ELISA-reader at 450 nm.

A calibration curve was constructed on a log-linear graph paper by plotting the mean absorbances of the five standards (0.5-10.5 µg/ml). The concentration of 8AA equivalents in each patient specimen were determined by interpolation on the calibration curve.

### Other markers

The urinary concentrations of Pyr were determined by HPLC technique. Briefly, the crosslinks are extracted from the hydrolysed urine sample by cellulose chromatography, separated by reversed-phase HPLC, and identified by spectrofluorometry. The area of the fluorescent peak was quantified by comparison with calibrated Pyr external standards purified from human cortical bone. The within-and total- assay coefficients of variations were less than 10% in the range of the standard curve (14.4 - 216 pmol, sample volume injected: 130 µL).

The urinary level of Hpr was measured by spectrophotometry. The within- and total-assay coefficients of variations were less than 13% in the range of the standard curve (38 - 305 µmol/L).

Values for the 8AA assay, Pyr and Hpr were divided by the corresponding creatinine value for normalization.

### Results

Figure 14 shows a schematic presentation of the location of the 8AA peptide selected for generating the antibodies used in the assay. The standards used in the assay define the measuring range from 0.5 to 10.5 mg/L. Around 5% of the urine samples tested had higher values than 10.5 mg/L. These samples were diluted 1+3 in Standard A and retested. The detection limit (defined as the concentration corresponding to the absorbency equal to the mean absorbency of the zero standard minus two SD_{Zero} Standard) is 0.2 mg/L.

For the technical validation the imprecision of the assay was calculated. Table 5 summarizes within- and total coefficients of variation. The data were calculated on the basis of four days of each six determinations of three urine samples using the same lot of the ELISA. The average within-run and total CVs were 5.3% and 6.6% respectively. Analytical recoveries, measured after adding increasing amounts of synthetic peptide to three different urine samples, averaged 100% (table 6).

The effect of dilution of samples with a high concentration in the ELISA procedure is shown in table 7. Samples were diluted in the Zero Standard (500 mM TRIS, 0.1% Tween 20, 0.1% BSA pH=8.0). The content in the undiluted urine sample was assigned the value of 100%. The overall average for different dilutions of the urine samples was 99%.

**Table 5.**

| Assay Imprecision | | | | |
|---|---|---|---|---|
| | Within-run | | Total | |
| Sample | Mean (mg/L) | CV(%) | Mean (mg/L) | CV(%) |
| A | 2.20 | 5.7 | 2.20 | 6.3 |
| B | 3.51 | 4.1 | 3.51 | 5.2 |
| C | 7.12 | 6.2 | 7.12 | 8.3 |

The data were calculated on the basis of four days of each six determinations of three urine samples using the same lot of the 8AA ELISA.

**Table 6.**

| Recovery of 8AA antigen added to urine samples (n=5) | | |
|---|---|---|
| Recovered 8AA | | |
| Added 8AA (mg/L) | Mean±SD (mg/L) | Mean±SD |
| 1.50 | 1.46 ± 0.14 | 97 ± 9 |
| 3.00 | 3.06 ± 0.19 | 102 ± 6 |
| 6.00 | 6.10 ± 0.34 | 102 ± 6 |

The 8AA concentration range in the urine sample was 0.61-3.44 µg/mL.

**Table 7.**

| Effect of dilution of three urine samples in 8AA ELISA, sample concentration and percent of 8AA in undiluted sample | | | | | | |
|---|---|---|---|---|---|---|
| | X | | Y | | Z | |
| Dilution | (mg/L) | % | (mg/L) | % | (mg/L) | % |
| neat | 6.64 | 100 | 8.36 | 100 | 9.23 | 100 |
| 1+1 | 3.45 | 104 | 4.06 | 97 | 4.10 | 89 |
| 1+3 | 1.68 | 101 | 2.30 | 110 | 2.11 | 91 |
| 1+7 | 0.73 | 88 | 0.16 | 111 | 1.22 | 106 |

Repeated freezing and thawing up to five times followed by measurement in the assay did not change the urine concentration significantly in five samples tested (percent of initial value after five freeze-thaw cycles: mean±SD, n=5: 98.5±4.5%). Furthermore, in another five samples the antigen measured was stable in urine without any additives for at least 7 days at 20° C (percent of initial value: mean±SD, n=5: 98.8±5.1%).

Figure 15 shows the individual 8AA ELISA values in two postmenopausal populations (early postmenopausal, n=245, late postmenopausal n=165) compared to the normal premenopausal range (mean ± 2 SD) shown by the hatched area (premenopausal range 250 ± 220 mg/mol Cr, n=102). Of the early postmenopausal women, 36.7% had values above the 2 SD limit of premenopausal women. The figure was 31.5% for the late postmenopausal group. The mean value for the early postmenopausal women was not different from that of the late postmenopausal (419 mg/mol vs. 412 mg/mol) and there was no trend towards a change in the values with increasing menopausal age.

Figure 16 shows the correlation between the 8AA ELISA and D-Pyr measured by HPLC. The correlation was highly significant yielding an r-value of 0.83.

Figure 17 shows the correlation between the ELISA and Hpr. Also, this correlation was highly significant and yielded an r-value of 0.78.

In the assay described the peptide sequence Glu-Lys-Ala-His-Asp-Gly-Gly-Arg specific for the C-telopeptide I chain of type I collagen was selected for the generation of antibodies (see figure 14). The reasons for selecting this part of the molecule are several. The sequence contains an important region for intermolecular cross-links. Furthermore, it has been suggested that the proximity of crosslinking residues and the compact structure of these molecules protect such peptides from further renal degradation. It is therefore anticipated that such peptide sequences arising from the partial degradation of mature type I collagen can be found in urine.

This experiment shows that the ELISA evaluated has a good performance in respect to precision, recovery, and dilution of urine samples (tables 5, 6 and 7). In respect to the long term handling and assaying of the urine samples it has been shown that the samples tested can be repeatedly frozen and thawed and that the antigen in these samples is stable for at least seven days at 20° C.

As can be seen from fig. 15 36.7% and 31.5%, respectively, of the women tested have elevated values (above mean+ 2SD). This finding also is in good correspondence with previous findings which estimate that around one third of all women experience an accelerated bone loss after the menopause.

D-Pyr is a well established marker of bone resorption. By comparing results obtained on parallel samples (n=214) in the D-Pyr assay (HPLC) and the 8AA ELISA a high correlation was found (r=0.83). This suggests that the 8AA ELISA reflects a metabolic process similar or parallel to what is reflected by pyridinoline. The correlation to another well established marker of bone resorption, Hpr (r=0.78), further indicates that the 8AA ELISA reflects bone resorption in vivo.

### EXAMPLE 9: USE OF THE CROSSLAPS ELISA AS A NEW MARKER OF

### BONE RESORPTION

Since increased bone resorption is the direct pathogenetic factor for increase bone loss, much attention has been directed towards finding a specific marker for bone resorption. The urinary excretion of calcium and hydroxyproline have for many years been used as indices of bone resorption, but neither are specific for bone metabolism.

This example evaluates the CrossLaps™ ELISA as a marker of bone resorption. Other markers for bone turnover are included in the evaluation for comparison purposes.

**Premenopausal women:** Serum and urine samples were analyzed from 104 healthy premenopausal women aged 30 - 50 years (mean ± 1SD: 39.1 ± 5.5), all of whom had a history of regular vaginal bleeding and none of whom were taking any medication known to influence the calcium metabolism.

**Postmenopausal women:** The postmenopausal women were part of two large double-blind placebo-controlled randomized studies. The main objective of the studies was to evaluate different preventive therapies for the early postmenopausal bone loss.

**Baseline values** were determined in 180 randomly selected healthy postmenopausal women, aged 45-55 years (mean ± 1SD: 50.6 ± 2.36), all of whom had a history of a natural menopause six months to three years previously (mean ± 1SD: 20.0 ± 10.4 months), and none of whom were taking any medication known to influence the calcium metabolism.

Longitudinal serum and urine samples were furthermore determined in 80 randomly selected women after 12 months' hormone replacement therapy and in 35 randomly selected women after 12 months' placebo therapy. The hormone regimens consisted of 2 mg oral estradiol combined with either 1 mg cyproterone acetate, 0.75 mg levonorgestrel, 1 mg desogestrel, or 10 mg medroxyprogesterone acetate. Since the groups responded similarly within the hormone and placebo therapy, the data were analyzed together. The 35 of these women who received placebo treatment were followed for two years with repeated forearm bone mass measurements every three month which allowed calculation of the rate of bone loss (% per year).

### Blood and urine samples

Blood samples were taken and the second morning urine collected between 8 and 10 a.m. after at least 8 hours fast. The samples were stored at - 20°C until analyzed.

The assay was carried out as described previously (example 6). The intra- and inter-assay-coefficients of variation were less than 6% and 8%, respectively, and the detection limit was 0.2 g/ml.

The urinary concentrations of pyridinoline (Pyr) and deoxypyridinoline (D-Pyr) were determined in a randomly selected subset of the samples, i.e. 81 samples in total (29 premenopausal and 52 postmenopausal samples) by HPLC technique. Briefly, the crosslinks are extracted from the hydrolyzed urine sample by cellulose chromatography, separated by reversed-phase HPLC, and identified by spectrofluorimetry. The area of the fluorescent peak was quantified by comparison with calibrated Pyr and D-Pyr external standards purified from human cortical bone. The intra- and inter-assay variations were less than 10% for Pyr and less than 15% for D-Pyr. The urinary level of hydroxyproline (Hpr) was measured by spectrophotometry, and serum levels of osteocalcin (osteocalcin) were measured by a radioimmunoassay (RIA).

Creatinine was measured on an auto-analyzer and the values were used to correct all urinary parameters (CrossLaps, Pyr, D-Pyr, Hpr). Bone mineral content (BMC) of the distal forearm was measured at baseline and every three months by single photon absorptiometry on a bone densitometer. The scanning is started where the distance between radius and ulna is 8 mm. From this point the scanning moves proximally for 24 mm. The long-term in-vivo precision is less than 1%. Bone mineral density (BMD) of the lumbar spine was measured either by dual energy photon or x-ray absorptiometry.

### Statistical analysis

The baseline values of CrossLaps and Pyr were correlated by linear regression analysis. In order to avoid introducing creatinine on both axis, the used values were not corrected for creatinine. Differences between premenopausal and postmenopausal groups were assessed by Student's t-test for unpaired data. T-scores were calculated as the postmenopausal deviation from the premenopausal mean values in premenopausal standard deviations. T-scores were compared by analysis of variance. The decreases from baseline in the different markers and bone mass during 12 months' hormone therapy were evaluated by Student's t-test for paired data. The changes in the placebo and the hormone groups were evaluated on a percentage scale. The individual baseline values were defined as 100% and all subsequent values were calculated as a percentage of the baseline value. The mean values of each group were compared by Student's t-test for unpaired data (rate of loss). The baseline values of CrossLaps were correlated to the rate of loss by linear regression analysis.

### Results

In **figure 18** the correlation between CrossLaps and Pyr is visualized (n=81). The r-value was 0.83.

**Figure 19** shows the individual values of CrossLaps/Cr, Hpr/Cr and osteocalcin in the pre- (N=104) and postmenopausal (n=180) women. The postmenopausal women had significantly increased values of all three markers of 71% (CrossLaps), 23% (Hpr) and 52% (osteocalcin). When the response to menopause was expressed in T-scores, CrossLaps yielded a T-score of 1.6, Hpr 0.7, and for osteocalcin 1.0 (p<0.001). In a randomly selected subset of the samples (premenopausal n=29 and postmenopausal n=28) the increases in per cent were 31% for Pyr and 50% for D-Pyr. The corresponding T-scores were 1.3 for Pyr as well as for D-Pyr, which was not significantly different from the T-score of CrossLaps (The T-score for CrossLaps was 1.5 in the same subgroup). Of the 180 postmenopausal women determined at baseline, 41% had Crosslaps values above 2 standard deviations above premenopausal mean value. The corresponding values for Hpr and osteocalcin were 12% and 23%, respectively.

**Figure 20** shows CrossLaps/Cr, Hpr/Cr, osteocalcin and forearm bone mass after 12 months' hormone replacement (n=80) or placebo (n=35) therapy expressed as a per cent of the baseline values. In all markers, there were statistically significant decreases in the hormone group compared to the placebo group. CrossLaps decreased by 60.7%, Hpr by 30.5%, and osteocalcin by 55.4%. In a randomly selected subset of the samples (hormone replacement n=12 and placebo n=12) the decrease in per cent were 22% for Pyr and 29% for D-Pyr. Forearm bone mass decreased by 3.1% and spinal bone mass with 3.1% per year.

**Figure 21** shows the correlation between baseline values of CrossLaps/Cr and the rate of loss (% per year) determined by forearm bone mass measurements over 24 months. The correlation yielded an r-value of -0.61 (p<0.001). The corresponding correlations for Hpr and osteocalcin yielded r-values of -0.46 and -0.44.

The above demonstrates that CrossLaps correlates highly significantly to pyridinoline which has been shown to be a sensitive marker of bone resorption. This indicates that CrossLaps also might be a sensitive marker of bone resorption. The samples in the present study represent a homogeneous group of women and the range of values is therefore relatively narrow. If patients with Paget's disease and extremely high bone turnover had been included, it might be anticipated that the correlation would have been even higher. The increase at menopause in CrossLaps was pronounced (more than 70%), indicating that it is a sensitive marker of the metabolic changes taking place at the menopause. The T-values may differ extensively with different study populations and should thus be interpreted with caution. However, for comparison of different markers the T-value is a valuable tool to give an indication of the sensitivity of the marker in question. The data of the present study thus indicates that CrossLaps is sensitive to the effect of menopause on calcium metabolism. The premenopausal women were not age-matched to the postmenopausal women. They were, however, on the average 40 years old and the impact of age, if any, must thus be marginal. Furthermore, in a comparative study like this the markers would most likely be influenced in a similar way.

The CrossLaps value decreased substantially in response to hormone replacement therapy, which opens the possibility that it might be a useful marker to monitor treatment efficacy. When compared to the markers normally used to follow-up on a hormone replacement therapy (osteocalcin, Hpr, Pyr, and D-pyr), CrossLaps performed better than the other resorption markers tested. The relatively small number of samples employed in the Pyr and D-Pyr study, however, limits the possibilities for more firm conclusions.

A Z-score calculated as the deviation of the active group from the placebo group in standard deviations is also higher for Crosslaps than for the other markers (1.5 for CrossLaps™, 0.7 for Hpr and 1.1 for osteocalcin; p<0.001).

It has been shown that the rate of the postmenopausal bone loss can be estimated by means of a panel of biochemical markers of bone turnover. The rate of loss would give the first indication of risk of osteoporosis, and could decrease the number that should be referred to a bone mass measurement. The correlation between CrossLaps and the rate of loss measured by single photon absorptiometry markers was found to be -0.61 (figure 21) which is higher than what has previously been found using other markers of bone turnover. Urine CrossLaps concentrations might thus be used to predict the rate of loss. If a bone loss of more than 3.0% per cent per year is defined to be a risk factor, the use of a CrossLaps cut off limit of 600 µg/mmol Cr in this population gives a diagnostic sensitivity of 73% and a specificity of 79%. A bone loss level of 3% per year was used to distinguish between "fast" and "slow" bone losers because it has earlier been found that this rate of loss is gives an appropriate separation in the bimodal bone loss distribution.

In this example, the rate of spinal bone loss was not determined with the required precision and accuracy, since many of the measurements were performed with DPA. The correlation to baseline CrossLaps is therefore not calculated.

In a 15 year follow-up of a group of healthy postmenopausal women it was shown that rate of loss is significantly related to the later incidence of fracture, indicating that rate of loss is a risk factor in itself. It is generally accepted that rapid changes are less well tolerated by the body than slow changes, even with the same outcome. This makes it even more important to identify individuals with an increased bone turnover. Seen in this context a specificity of almost 80% and a sensitivity of more than 70% makes the method of the invention a potential useful screening parameter in the risk assessment of postmenopausal osteoporosis.

The invention has been described above by reference to specific embodiments. It will be apparent to those skilled in the art, however, that many additions, deletions and modifications are possible without departing from the invention as claimed below.

### APPENDIX A

Collagen alpha 1(I) chain precursor - Human (fragments) Homo sapiens (man)

Number of residues = 1341

5 10 15 20 25 30
1 M F S F V D L R L L L L L A A T A L L T H G Q E E G Q V E G
31 Q D E D I P P I T C V Q N G L R Y H D R D V W K P E P C Q I
61 C V C D N G K V L C D D V I C D E T K N C P G A E V P E G E
91 C C P V C P D G S E S P T D Q E T T G V E G P K G D T G P R
121 G P R G P A G P P G R D G I P G Q P G L P G P P G P P G P P
151 G P P G L G G N F A P Q L S Y G Y D E K S T G G I S V P G P
181 M(G P S G P R G L P G P P G A P G P Z G F Z G P P G Z P G Z
211 P G A S G P)M(G P R G P P G P P G K B G B B G Z A G K P G R
241 P G Z R G P P G P Z G A R G L P G T A G L P)G M K G H R(G F
271 S G L B G A K G B A G P A G P K G Z P G S P G Z B G A P G Z)
301 M/G P P G P K G N S G E P G A P G S K G D T G A K G E P G P
331 V G V Q G P P G P A G E E G K R G A R G E P G P T G L P G P
361 P G E R G G P G S R G F P G A D G V A G P K G P A G E R G S
391 P G P A G P K G S P G E A G R P G E A G L P G A K G L T G S
421 P G S P G P D G K T G P P G P A G Q D G R P G P P G P P G A
451 R G Q A G V M G F P G P K G A A G E P G K A G E R G V P G P
481 P G A V G P A G K D G E A G A Q G P P G P A G P A G E R G E
511 Q G P A G S P G F Q G L P G P A G P P G E A G K P G E Q G V
541 P G D L G A P G P S G A R G E R G F P G E R G V Q G P P G P
571 A G P R G A N G A P G N D G A K G D A G A P G A P G S Q G A
601 P G L Q G M P G E R G A A G L P G P K G D R G D A G P K G A
631 D G S P G K D G V R G L T G P I G P P G P A G A P G D K G E
661 S G P S G P A G P T G A R G A P G D R G E P G P P G P A G F
691 A G P P G A D G Q P G A K G E P G D A G A K G D A G P P G P
721 A G P A G P P G P I G N V G A P G A K G A R G S A G P P G A
751 T G F P G A A G R V G P P G P S G N A G P P G P P G P A G K
781 E G G K G P R G E T G P A G R P G E V G P P G P P G P A G E
811 K G S P G A D G P A G A P G T P G P Q G I A G Q R G V V G L
841 P G Q R G E R G F P G L P G P S G E P G K Q G P S G A S G E
871 R G P P G P M G P P G L A G P P G E S G R E G A P G A E G S
901 P G R D G S P G A K G D R G E T G P A G P P G A X G A X G A
931 P G P V G P A G K S G D R G E T G P A G P A G P V G P A G A
961 R G P A G P Q G P R G D K G E T G E Q G D R G I K G H R G F
991 S G L Q G P P G P P G S P G E Q G P S G A S G P A G P R G P
1021 P G S A G A P G K D G L N G L P G P I G P P G P R G R T G D
1051 A G P V G P P G P P G P P G P P G P P S A G F D F S F L P Q
1081 P P Q E K A H D G G R Y Y R A D D A N V V R D R D L E V D T
1111 T L K S L S Q Q I E N I R S P E G X R K N P A R T C R D L K
1141 M C H S D W K S G E Y W I D P N Q G C N L D A I K V F C N M
1171 E T G E T C V Y P T Q P S V A Q K N W Y I S K N P K D K R H
1201 V W F G E S M T D G F Q F E Y G G Q G S D P A D V A I Q L T
1231 F L R L M S T E A S Q N I T Y H C K N S V A Y M D Q Q T G N
1261 L K K A L L L X G S N E I E I R A E G N S R F T Y S V T V D
1291 G C T S H T G A W G K T V I E Y K T T K S S R L P I I D V A
1321 P L D V G A P D Q E F G F D V G P V C F L

### Sequence of collagen alpha 1(I) chain precursor prepared from:

Chu, M.L., de Wet, W., Bernard, M., Ding, J.F., Morabito, M., Myers, J., Williams, C., and Ramirez, F., Nature 310, 337-340, 1984 (Human, sequence of residues 1-181 translated from the DNA sequence)
Click, E.M., and Bornstein, P., Biochemistry 9, 4699-4706, 1970 (Human skin, CNBr0-1, CNBr2, CNBr4, CNBr5, partial sequence of residues 162-301)
Morgan, P.H., Jacobs, H.G., Segrest, J.P., and Cunningham, L.W., J. Biol. Chem. 245, 5042-5048, 1970 (Human skin, sequence of residues 263-268)
Bernard, M.P., Chu, M.L., Myers, J.C., Ramirez, F., Eikenberry, E.F., and Prockop, D.J., Biochemistry 22, 5213-5223, 1983 (Sequence of residues 302-1341 translated from the mRNA sequence)

Procollagen alpha 2(I) chain precursor Homo sapiens (man) 
Number of residues = 1366

5 10 15 20 25 30
1 M L S F V D T R T L L L L A V T L C L A T C Q S L Q E E T V
31 R K G P A G D R G P R G E R G P P G P P G R D G E D G P T G
61 P P G P P G P P G P P G L G G N F A A Q Y D G K G V G L G P
91 G P M G L M G P R G P P G A A G A P G P Q G F Q G P A G E P
121 G E P G Q T G P A G A R G P A G P P G K A G E D G H P G K P
151 G R P G E R G V V G P Q G A R G F P G T P G L P G F K G I R
181 G H N G L D G L K G Q P G A P G V K G E P G A P G E N G T P
211 G Q T G A R G L P G E R G R V G A P G P A G A R G S D G S V
241 G P V G P A G P N G S A G P P G F P G A P G P K G E I G A V
271 G N A G P T G P A G P R G E V G L P G L S G P V G P P G N P
301 G A N G L T G A K G A A G L P G V A G A P G L P G P R G I P
331 G P P G A A G T T G A R G L V G E P G P A G S K G E S G N K
361 G E P G S A G P Q G P P G P S G E E G K R G P N G E A G S A
391 G P P G P P G L R G S P G S R G L P G A D G R A G V M G P P
421 G S R G A S G P A G V R G P N G D A G R P G E P G L M G P R
451 G L P G S P G N I G P A G K E G P V G L P G I D G R P G P I
481 G P V G A R G E P G N I G F P G P K G P T G D P G K N G D K
511 G H A G L A G A R G A P G P D G N N G A Q G P P G P Q G V Q
541 G G K G E Q G P A G P P G F Q G L P G P S G P A G E V G K P
571 G E R G L H G E F G L P G P A G P R G E R G P P G E S G A A
601 G P T G P I G S R G P S G P P G P D G N K G E P G V V G A V
631 G T A G P S G P S G L P G E R G A A G I P G G K G E K G E P
661 G L R G E I G N P G R D G A R G A H G A V G A P G P A G A T
691 G D R G E A G A A G P A G P A G P R G S P G E R G E V G P A
721 G P N G F A G P A G A A G Q P G A K G E R G G K G P K G E N
751 G V V G P T G P V G A A G P A G P N G P P G P A G S R G D G
781 G P P G M T G F P G A A G R T G P P G P S G I S G P P G P P
811 G P A G K E G L R G P R G D Q G P V G R T G E V G A V G P P
841 G F A G E K G P S G E A G T A G P P G T P G P Q G L L G A P
871 G I L G L P G S R G E R G L P G V A G A V G E P G P L G I A
901 G P P G A R G P P G A V G S P G V N G A P G E A G R D G N P
931 G N D G P P G R D G Q P G H K G E R G Y P G N I G P V G A A
961 G A P G P H G P V G P A G K H G N R G E T G P S G P V G P A
991 G A V G P R G P S G P Q G I R G D K G E P G E K G P R G L P
1021 G F K G H N G L Q G L P G I A G H H G D Q G A P G S V G P A
1051 G P R G P A G P S G P A G K D G R T G H P G T V G P A G I R
1081 G P Q G H Q G P A G P P G P P G P P G P P G V S G G G Y D F
1111 G Y D G D F Y R A D Q P R S A P S L R P K D Y E V D A T L K
1141 S L N N Q I E T L L T P E G S R K N P A R T C R D L R L S H
1171 P E W S S G Y Y W I D P N Q G C T M E A I K V Y C D F P T G
1201 E T C I R A Q P E N I P A K N W Y R S S K D K K H V W L G E
1231 T I N A G S Q F E Y N V E G V T S K E M A T Q L A F M R L L
1261 A N Y A S Q N I T Y H C K N S I A Y M D E E T G N L K K A V
1291 I L Q G S N D V E L V A E G N S R F T Y T V L V D G C S K K
1321 T N E W G K T I I E Y K T N K P S R L P F L D I A P L D I G
1351 G A D H E F F V D I G P V C F K
 Sequence of procollagen alpha 2(I) chain precursor prepared from:
de Wet, W., Bernard, M., Benson-Chanda, V., Chu, M.L., Dickson, L., Weil, D., and Ramirez, F., J. Biol. Chem. 262, 16032-16036, 1987 (Sequence translated from the mRNA sequence)
Title: Organization of the human pro-alpha-2(I) collagen gene.

Collagen alpha 1(II) chain precursor Homo sapiens (man)

Number of residues = 1418

1 MIRLGAPQSL VLLTLLVAAV LRCQGQDVRQ PGPKGQKGEP GDIKDIVGPK
51 GPPGPQGPAG EQGPRGDRGD KGEKGAPGPR GRDGEPGTLG NPGPPGPPGP
101 PGPPGLGGNF AAQMAGGFDE KAGGAQLGVM QGPMGPMGPR GPPGPAGAPG
151 PQGFQGNPGE PGEPGVSGPM GPRGPPGPPG KPGDDGEAGK PGKAGERGPP
201 GPQGARGFPG TPGLPGVKGH RGYPGLDGAK GEAGAPGVKG ESGSPGENGS
251 PGPMGPRGLP GERGRTGPAG AAGARGNDGQ PGPAGPPGPV GPAGGPGFPG
301 APGAKGEAGP TGARGPEGAQ GPRGEPGTPG SPGPAGASGN PGTDGIPGAK
351 GSAGAPGIAG APGFPGPRGP PDPQGATGPL GPKGQTGKPG IAGFKGEQGP
401 KGEPGPAGPQ GAPGPAGEEG KRGARGEPGG VGPIGPPGER GAPGNRGFPG
451 QDGLAGPKGA PGERGPSGLA GPKGANGDPG RPGEPGLPGA RGLTGRPGDA
501 GPQGKVGPSG APGEDGRPGP PGPQGARGQP GVMGFPGPKG ANGEPGKAGE
551 KGLPGAPGLR GLPGKDGETG AEGPPGPAGP AGERGEQGAP GPSGFQGLPG
601 PPGPPGEGGK PGDQGVPGEA GAPGLVGPRG ERGFPGERGS PGAQGLQGPR
651 GLPGTPGTDG PKGASGPAGP PGAQGPPGLQ GMPGERGAAG IAGPKGDRGD
701 VGEKGPEGAP GKDGGRGLTG PIGPPGPAGA NGEKGEVGPP GPAGSAGARG
751 APGERGETGP PGTSGIAGPP GADGQPGAKG EQGEAGQKGD AGAPGPQGPS
801 GAPGPQGPTG VTGPKGARGA QGPPGATGFP GAAGRVGPPG SNGNPGPPGP
851 PGPSGKDGPK GARGDSGPPG RAGEPGLQGP AGPPGEKGEP GDDGPSGAEG
901 PPGPQGLAGQ RGIVGLPGQR GERGFPGLPG PSGEPGQQGA PGASGDRGPP
951 GPVGPPGLTG PAGEPGREGS PGADGPPGRD GAAGVKGDRG ETGAVGAPGA
1001 PGPPGSPGPA GPTGKQGDRG EAGAQGPMGP SGPAGARGIQ GPQGPRGDKG
1051 EAGEPGERGL KGHRGFTGLQ GLPGPPGPSG DQGASGPAGP SGPRGPPGPV
1101 GPSGKDGANG IPGPIGPPGP RGRSGETGPA GPPGNPGPPG PPGPPGPGID
1151 MSAFAGLGPR EKGPDPLQYM RADQAAGGLR QHDAEVDATL KSLNNQIESI
1201 RSPEGSRKNP ARTCRDLKLC HPEWKSGDYW IDPNQGCTLD AMKVFCNMET
1251 GETCVYPNPA NVPKKNWWSS KSKEKKHIWF GETINGGFHF SYGDDNLAPN
1301 TANVQMTFLR LLSTEGSQNI TYHCKNSIAY LDEAAGNLKK ALLIQGSNDV
1351 EIRAEGNSRF TYTALKDGCT KHTGKWGKTV IEYRSQKTSR LPIIDIAPMD
1401 IGGPEQEFGV DIGPVCFL

### Sequence of collagen alpha 1(II) chain precursor prepared from:

Su, M.W., Lee, B., Ramirez, F., Machado, M., and Horton, W. Nucleic Acids Res. 17, 9473, 1989
   Nucleotide sequence of the full length cDNA encoding for human type II procollagen.
Baldwin, C.T., Reginato, A.M., Smith, C., Jimenez, S.A., and Prockop, D.J., Biochem. J. 262, 521-528, 1989
   Structure of cDNA clones coding for human type II procollagen. The alpha-1(II) chain is more similar to the alpha-1(I) chain than two other alpha chains of fibrillar collagens.
Ala-Kokko, L., Baldwin, C.T., Moskowitz, R.W., and Prockop, D.J. Proc. Natl. Acad. Sci. U.S.A. 87, 6565-6568, 1990
   Single base mutation in the type II procollagen gene (COL2A1) as a cause of primary osteoarthritis associated with a mild chondrodysplasia.
Ramirez, F.
   submitted to the EMBL Data Library, December 1988 Reference number: S04892
Vikkula, M., and Peltonen, L.
   FEES Lett. 250, 171-174, 1989
   Structural analyses of the polymorphic area in type II collagen gene.

Collagen alpha 1(III) chain precursor Homo sapiens (man)

Number of residues = 1078

5 10 15 20 25 30
1/Q N Y S P Q Y D S Y D V K S G G V A V G G L A G Y P G P A G
31 P P G P P G P P G T S G H P G S P G S P G Y Q G P P G E P G
61 Q A G P S G P P G P P G A I G P S G P A G K D G E S G R P G
91 R P G D R G L P G P P G I K G P A G I P G F P G M K G H R G
121 F D G R N G E K G E T G A P G L K G E N G L P G E N G A P G
151 P M G P R G A P G E R G R P G L P G A A G A R G N D G A R G
181 S D G Q P G P P G P P G T A G F P G S P G A K G E V G P A G
211 S P G S N G A P G Q R G E P G P Q G H A G A Q G P P G P P G
241 I N G S P G G K G E M G P A G I P G A P G L M G A R G P P G
271 P A G A N G A P G L R G G A G E P G K N G A K G E P G P R G
301 E R G E A G I P G V P G A K G E D G K D G S P G D P G A N G
331 L P G A A G E R G A L G S R G P A G P N G I P G E K G P A G
361 E R G A P G P A G P R G A A G E P G R D G V P G G P G M R G
391 M P G S P G G P G S D G K P G P P G S Q G E S G R P G P P G
421 P S G P R G Q P G V M G F P G P K G N D G A P G K N G E R G
451 G P G G P G P Q G P P G K N G E Y G P Q G P P G P T G P G G
481 D K G D T G P R G P Q G L Q G L P G T G G P P G E N G K P G
511 E P G P K G E A G A P G A P G G K G D A G A P G E R G P P G
541 L A G A P G L R G G A G P P G P E G G K G A A G P P G P P G
571 A A G T P G L Q G M P G E R G G L G S P G P K G D K G E P G
601 G P G A D G V P G K D G P R G P T G P I G P P G P A G Q P G
631 D K G E G G A P G L P G I A G P R G S P G E R G E T G P P G
661 P A G F P G A P G Q N G E P G G K G E R G A P G E K G E G G
691 P P G V A G P P G G S G P A G P P G P Q G V K G E R G S P G
721 G P G A A G F P G A R G L P G P P G S N G N P G P P G P S G
751 S P G K D G P P G P A G N T G A P G S P G V S G P K G D A G
781 Q P G E K G S P G A Q G P P G A P G P L G I A G I T G A R G
811 L A G P P G M P G P R G S P G P Q G V K G E S G K P G A N G
841 L S G E R G P P G P Q G L P G L A G T A G E P G R D G N P G
871 S D G L P G R D G S P G G K G D R G E N G S P G A P G A P G
901 H P G P P G P V G P A G K S G D R G E S G P A G P A G A P G
931 P A G S R G A P G P Q G P R G D K G E T G E R G A A G I K G
961 H R G F P G N P G A P G S P G P A G Q Q G A I G S P G P A G
991 P R G P V G P S G P P G K D G T S G H P G P I G P P G P R G
1021 N R G E R G S E G S P G H P G Q S G P P G P P G A P G P C C
1051 G G V G A P A I A G I G A E K A G G F A P Y Y G D E P M/

### Sequence of collagen alpha 1(III) chain precursor prepared from:

Janeczko, R.A., and Ramirez, F., Nucleic Acids Res. 17, 6742, 1989 Nucleotide and amino acid sequences of the entire human alpha-1 (III) collagen.

## Claims

1. A method of assay for collagen Type 1 fragments in a body fluid, in which collagen fragments in a said body fluid and a synthetic peptide having a sequence derived from collagen and having a potential site for cross-linking but containing no collagen crosslink structure immobilised on a solid surface are contacted with an immunological binding partner which is immunoreactive with the immobilised synthetic peptide and with collagen fragments in said body fluid, wherein the immunological binding partner has been raised against a peptide consisting of the amino acid sequence Gln-Tyr-Asp-Gly-Lys-Gly-Val-Gly and is immuno-reactive with an immobilised peptide consisting of the amino acid sequence Gln-Tyr-Asp-Gly-Lys-Gly-Val-Gly.

2. A method as claimed in Claim 1, comprising:
(a) contacting a sample of the body fluid with a synthetic peptide immobilised on a solid support and with an immunological binding partner which is immunologically reactive with said immobilised synthetic peptide, and which has been raised against a peptide consisting of the amino acid sequence Gln-Tyr-Asp-Gly-Lys-Gly-Val-Gly, wherein the collagen fragments compete with said synthetic peptide for binding with the immunological binding partner, and
(b) quantifying the amount of collagen fragments in the body fluid by measuring the amount of binding of said immunological binding partner with said synthetic peptide;
wherein
said immunological binding partner is immunologically reactive with a peptide immobilised on a solid support and consisting of the amino acid sequence Gln-Tyr-Asp-Gly-Lys-Gly-Val-Gly; and
said immobilised synthetic peptide is derived from collagen, contains no collagen cross-link structure but contains a potential site for cross-linking and is immunologically reactive with said immunological binding partner.

3. A method as claimed in Claim 2, wherein said immobilised synthetic peptide consists of fewer than 10 amino acids.

4. A method as claimed in Claim 2 or Claim 3, wherein the immobilised synthetic peptide has the amino acid sequence Gln-Tyr-Asp-Gly-Lys-Gly-Val-Gly or has said sequence Gln-Tyr-Asp-Gly-I,ys-Gly-Val-Gly modified by the addition or omission of one or more amino acids whilst retaining the ability to raise antibodies recognising the corresponding native collagen fragment or to inhibit the binding of such antibodies to the corresponding native collagen fragment.

5. A method as claimed in any preceding claim, wherein said immunological binding partner is a whole antibody.

6. A method as claimed in Claim 5, wherein said antibody is a monoclonal antibody.

## Patentansprüche

1. Analytisches Testverfahren zum Messen von Fragmenten des Kollagens vom Typ I in einer Körperflüssigkeit, bei dem Fragmente des Kollagens in der genannten Körperflüssigkeit sowie ein synthetisches Peptid, welches eine Sequenz aufweist, die sich von Kollagen ableitet und eine potentielle Vernetzungsstelle enthält - jedoch keine Quervernetzung in Form einer Kollagenstruktur aufweist und auf einer festen Fläche immobilisiert ist, miteinander zusammen mit einem immunologischen Bindungspartner in Kontakt gebracht werden, wobei dieser Bindungspartner immunoreaktiv mit dem immobilisierten synthetischen Peptid und den Fragmenten des Kollagens in der genannten Körperflüssigkeit ist, worin der immunologische Bindungspartner gegen ein aus der Aminosäuresequenz Gln-Tyr-Asp-Gly-Lys-Gly-Val-Gly bestehendes Peptid erzeugt wurde und mit einem immobilisierten Peptid immunreaktiv ist, das aus der Aminosäuresequenz Gln-Tyr-Asp-Gly-Lys-Gly-Val-Gly besteht.

2. Verfahren nach Anspruch 1, das die folgende Maßnahme umfasst:
a) eine Probe der Körperflüssigkeit mit einem synthetischen Peptid, das auf einem festen Träger immobilisiert ist, zusammen mit einem immunologischen Bindungspartner in Kontakt zu bringen, wobei dieser Bindungspartner immunoreaktiv mit dem genannten immobilisierten, synthetischen Peptid ist, wobei jener gegen ein aus der Aminosäuresequenz Gln-Tyr-Asp-Gly-Lys-Gly-Val-Gly bestehendes Peptid erzeugt wurde, wobei die Fragmente des Kollagens mit dem genannten synthetischen Peptid um die Bindung mit dem immunologischen Reaktionspartner in Konkurrenz treten, sowie die Maßnahme
b) die Menge an Fragmenten des Kollagens in der Körperflüssigkeit durch die Messung des Anteils der Bindung an dem genannten immunologischen Bindungspartner mit dem genannten synthetischen Peptid quantitativ zu bestimmen,
wobei in dem Verfahren der genannte immunologische Bindungspartner immunoreaktiv mit einem Peptid ist, das auf einem festen Träger immobilisiert ist und aus der Aminosäuresequenz Gln-Tyr-Asp-Gly-Lys-Gly-Val-Gly besteht;
und wobei das genannte immobilisierte synthetische Peptid von Kollagen abgeleitet ist, das zwar keine Quervernetzung in Form einer Kollagenstruktur aufweist, jedoch eine potentielle Vernetzungsstelle aufweist, wobei dieses Peptid mit dem genannten immunologischen Bindungspartner immunologisch reaktiv ist.

3. Verfahren nach Anspruch 2, bei dem das immobilisierte synthetische Peptid aus weniger als 10 Aminosäuren besteht.

4. Verfahren nach Anspruch 2 oder Anspruch 3, bei dem das immobilisierte synthetische Peptid die Aminosäuresequenz Gln-Tyr-Asp-Gly-Lys-Gly-Val-Gly aufweist oder die genannte Sequenz Gln-Tyr-Asp-Gly-Lys-Gly-Val-Gly besitzt, die durch das Hinzufügen oder die Weglassung einer oder mehrerer Aminosäuren modifiziert worden ist, wobei jenes Peptid jedoch in diesem Falle die Fähigkeit, Antikörper zu erzeugen, die das entsprechende native Fragment des Kollagens erkennen, oder die Fähigkeit, die Bindung derartiger Antikörper an das entsprechende native Fragment des Kollagens zu unterdrücken, beibehalten hat.

5. Verfahren nach einem der vorstehenden Ansprüche, bei dem der immunologische Bindungspartner in Form eines vollständigen Antikörpers vorliegt.

6. Verfahren nach Anspruch 5, bei dem der Antikörper in Form eines monoklonalen Antikörpers vorliegt.

## Revendications

1. Procédé de dosage pour des fragments de collagène de type I dans un fluide corporel, dans lequel les fragments de collagène dans ledit un fluide corporel et un peptide synthétique ayant une séquence dérivée du collagène et ayant un site potentiel pour une réticulation mais ne contenant aucune structure réticulant avec le collagène immobilisé sur une surface solide sont mis en contact avec un partenaire de liaison immunologique qui est immunoréactif avec le peptide synthétique immobilisé et avec les fragments de collagène dans ledit fluide corporel, dans lequel le partenaire de liaison immunologique qui a été conçu par rapport à un peptide constitué de la séquence d'acides aminés Gln-Tyr-Asp-Gly-Lys-Gly-Val-Gly est immunoréactif avec un peptide immobilisé composé de la séquence d'acides aminés Gln-Tyr-Asp-Gly-Lys-Gly-Val-Gly.

2. Procédé selon la revendication 1, consistant:
(a) à mettre en contact un échantillon du fluide corporel avec un peptide synthétique immobilisé sur un support solide et avec un partenaire de liaison immunologique qui est immunologiquement réactif avec ledit peptide synthétique immobilisé, et qui a été conçu par rapport à une peptide constitué de la séquence d'acides aminés Gln-Tyr-Asp-Gly-Lys-Gly-Val-Gly, dans lequel les fragments de collagène concurrencent ledit peptide synthétique pour se lier au partenaire de liaison immunologique; et
(b) à quantifier la quantité de fragments de collagène dans le fluide corporel en mesurant la quantité de liaison dudit partenaire de liaison immunologique avec ledit peptide synthétique;
dans lequel
ledit partenaire de liaison immunologique est immunologiquement réactif avec un peptide immobilisé sur un support solide et composé de la séquence d'acides aminés Gln-Tyr-Asp-Gly-Lys-Gly-Val-Gly; et
ledit peptide synthétique immobilisé est dérivé du collagène, ne contient aucune structure réticulant le collagène mais contient un site potentiel pour la réticulation, et est immunologiquement réactif avec ledit partenaire de liaison immunologique.

3. Procédé selon la revendication 2, dans lequel ledit peptide synthétique immobilisé se compose de moins de 10 acides aminés.

4. Procédé selon la revendication 2 ou 3, dans lequel le peptide synthétique immobilisé possède la séquence d'acides aminés Gln-Tyr-Asp-Gly-Lys-Gly-Val-Gly ou possède ladite séquence Gln-Tyr-Asp-Gly-Lys-Gly-Val-Gly modifiée par l'addition ou l'omission d'un ou plusieurs acides aminés tout en conservant la capacité à élever des anticorps reconnaissants le fragment de collagène naturel correspondant ou à inhiber la liaison de tels anticorps au fragment de collagène naturel correspondant.

5. Procédé selon l'une quelconque des revendications précédentes, dans lequel ledit partenaire de liaison immunologique est un anticorps entier.

6. Procédé selon la revendication 5, dans lequel ledit anticorps est un anticorps monoclonal.
